(19) Europäisches Patentamt
European Patent Office
Office européen des brevets

(11) EP 1 852 112 A1

(12) **EUROPEAN PATENT APPLICATION**
published in accordance with Art. 158(3) EPC

(43) Date of publication:
**07.11.2007 Bulletin 2007/45**

(21) Application number: **06714136.6**

(22) Date of filing: **21.02.2006**

(51) Int Cl.:
***A61K 31/12*** (2006.01) ***A61K 31/165*** (2006.01)
***A61K 31/216*** (2006.01) ***A61K 31/343*** (2006.01)
***A61K 31/36*** (2006.01) ***A61K 31/381*** (2006.01)
***A61K 31/40*** (2006.01) ***A61K 31/4015*** (2006.01)
***A61K 31/4406*** (2006.01) ***A61K 31/445*** (2006.01)
***A61K 31/4535*** (2006.01) ***A61K 31/472*** (2006.01)
***A61K 31/495*** (2006.01) ***A61K 31/496*** (2006.01)
***A61K 31/506*** (2006.01) ***A61K 31/5375*** (2006.01)
***A61K 31/5377*** (2006.01) ***A61P 35/00*** (2006.01)
***A61P 35/02*** (2006.01)

(86) International application number:
**PCT/JP2006/302996**

(87) International publication number:
**WO 2006/088193 (24.08.2006 Gazette 2006/34)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.02.2005 JP 2005044845**

(71) Applicant: **KYOWA HAKKO KOGYO CO., LTD.**
**Chiyoda-ku,**
**Tokyo 100-8185 (JP)**

(72) Inventors:
- **KANDA, Yutaka**
  **c/o Pharmaceutical Research Center**
  **Sunto-gun, Shizuoka (JP)**
- **SOGA, Shiro**
  **c/o Pharmaceutical Research Center**
  **Sunto-gun, Shizuoka (JP)**
- **NAKASHIMA, Takayuki**
  **c/o Pharmaceutical Research Center**
  **Sunto-gun, Shizuoka (JP)**
- **NARA, Shinji**
  **c/o Pharmaceutical Research Center**
  **Sunto-gun, Shizuoka (JP)**
- **NAKAGAWA, Hiroshi**
  **c/o Pharmaceutical Research Center**
  **Sunto-gun, Shizuoka (JP)**
- **SHIOTSU, Yukimasa**
  **c/o Pharmaceutical Research Center**
  **Sunto-gun, Shizuoka (JP)**

(74) Representative: **Casalonga, Axel et al**
**Bureau Casalonga & Josse**
**Bayerstrasse 71/73**
**80335 München (DE)**

(54) **ANTI-TUMOR AGENT**

(57) The present invention provides a therapeutic agent for a tumor selected from a hematopoietic tumor and a solid tumor which comprises, as an active ingredient, a benzoyl compound represented by General Formula (I):

EP 1 852 112 A1

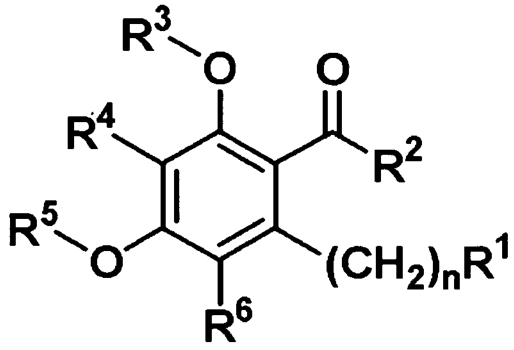

( I )

(wherein
n represents an integer of 1 to 5;
$R^1$ represents substituted or unsubstituted lower alkoxy, substituted or unsubstituted lower alkoxycarbonyl, $CONR^7R^8$ or the like;
$R^2$ represents substituted or unsubstituted aryl or a substituted or unsubstituted aromatic heterocyclic group;
$R^3$ and $R^5$ may be the same or different, and each represents a hydrogen atom, substituted or unsubstituted lower alkyl or the like;
$R^4$ represents a hydrogen atom, hydroxy or halogen; and
$R^6$ represents a hydrogen atom, halogen, substituted or unsubstituted lower alkyl or the like), a prodrug thereof or a pharmaceutically acceptable salt thereof.

## Description

Technical Field

The present invention relates to a therapeutic agent for a tumor comprising, as an active ingredient, a benzoyl compound, a prodrug thereof, or a pharmaceutically acceptable salt thereof.

Background Art

As a benzoyl compound having an antitumor activity, the following Compound A has been known (refer to Patent Document 1).

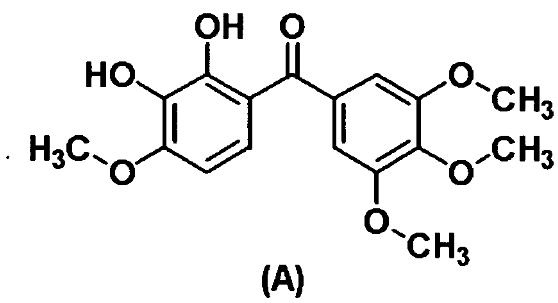

(A)

Also, benzoyl compounds having binding activity to heat shock protein 90 (Hsp90) family have been known (refer to Patent Document 2).

Patent Document 1: W02001/81288
Patent Document 2: W02005/000778

Disclosure of the Invention

Problems to be Solved by the Invention

An object of the present invention is to provide a therapeutic agent for a tumor selected from a hematopoietic tumor and a solid tumor which comprises, as an active ingredient, a benzoyl compound, a prodrug thereof or a pharmaceutically acceptable salt thereof.

Means for Solving the Problems

The present invention relates to the following (1) to (21).

(1) A therapeutic agent for a tumor selected from a hematopoietic tumor and a solid tumor which comprises, as an active ingredient, a benzoyl compound represented by General Formula (I):

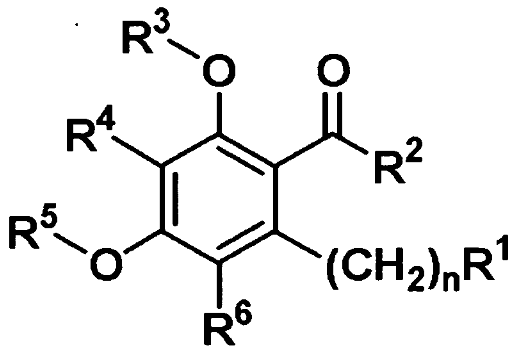

( I )

[wherein

n represents an integer of 1 to 5;

$R^1$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocyclic alkyl, substituted or unsubstituted aryl,

$CONR^7R^8$ (wherein $R^7$ and $R^8$ may be the same or different, and each represents a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocyclic alkyl, or substituted or unsubstituted aroyl, or $R^7$ and $R^8$ are combined together with the adjacent nitrogen atom thereto to form a substituted or unsubstituted heterocyclic group), or

$NR^9R^{10}$ (wherein $R^9$ and $R^{10}$ have the same meanings as the above $R^7$ and $R^8$, respectively);

$R^2$ represents substituted or unsubstituted aryl,

or

a substituted or unsubstituted aromatic heterocyclic group;

$R^3$ and $R^5$ may be the same or different, and each represents a hydrogen atom,

substituted or unsubstituted lower alkyl,

substituted or unsubstituted lower alkenyl,

substituted or unsubstituted lower alkanoyl,

substituted or unsubstituted cycloalkyl,

substituted or unsubstituted aralkyl,

or

substituted or unsubstituted aroyl;

$R^4$ represents a hydrogen atom,

hydroxy,

or

halogen; and

$R^6$ represents a hydrogen atom,

halogen,

cyano,

nitro,

substituted or unsubstituted lower alkyl,

substituted or unsubstituted lower alkenyl,

substituted or unsubstituted lower alkynyl,

substituted or unsubstituted lower alkoxy,

substituted or unsubstituted cycloalkyl,

amino,

lower alkylamino,

di(lower alkyl)amino,

carboxy,

substituted or unsubstituted lower alkoxycarbonyl,

substituted or unsubstituted lower alkanoyl,

substituted or unsubstituted aryloxy,

substituted or unsubstituted aryl,
a substituted or unsubstituted heterocyclic group,
substituted or unsubstituted aralkyl,
or
substituted or unsubstituted heterocyclic alkyl; with the proviso that

(i) when $R^3$ and $R^5$ are methyl and $R^4$ and $R^6$ are hydrogen atoms, and

(a) when $-(CH_2)_nR^1$ is methoxycarbonylmethyl,
$R^2$ is not a group selected from 2,4,6-trimethoxy-5-methoxycarbonyl-3-nitrophenyl, 3-cyano-2,4,6-trimethoxyphenyl, 5-cyano-2-ethoxy-4,6-dimethoxy-3-nitrophenyl, 2,6-dimethoxyphenyl, 2-chloro-6-methoxyphenyl and 2-chloro-4,6-dimethoxy-5-methoxycarbonyl-3-nitrophenyl,
(b) when $-(CH_2)_nR^1$ is ethoxycarbonylmethyl,
$R^2$ is not 2,4,6-trimethoxy-3-methoxycarbonylphenyl, and
(c) when $-(CH_2)_nR^1$ is N,N-dimethylaminomethyl,
$R^2$ is not phenyl,

(ii) when $R^3$, $R^4$, $R^5$ and $R^6$ are hydrogen atoms, and

(a) when $-(CH_2)_nR^1$ is 2-(acetoxymethyl)heptyl, 3-oxopentyl or pentyl,
$R^2$ is not 6-hydroxy-4-methoxy-3-methoxycarbonyl-2-pentylphenyl,
(b) when $-(CH_2)_nR^1$ is 3-oxopentyl,
$R^2$ is not a group selected from 3-benzyloxycarbonyl-6-hydroxy-4-methoxy-2-pentylphenyl and 3-carboxy-6-hydroxy-4-methoxy-2-pentylphenyl, and
(c) when $-(CH_2)_nR^1$ is n-propyl,
$R^2$ is not 2,4-dihydroxy-6-[(4-hydroxy-2-oxopyran-6-yl)methyl]phenyl,

(iii) when $R^3$ and $R^4$ are hydrogen atoms, $R^5$ is methyl, $R^6$ is methoxycarbonyl and $-(CH_2)_nR^1$ is pentyl,
$R^2$ is not a group selected from 6-[2-(acetoxymethyl)heptyl]-2,4-dihydroxyphenyl, 2,4-dihyroxy-6-pentylphenyl and 2,4-dihydroxy-6-(3-oxopentyl)phenyl,
(iv) when $R^3$ and $R^5$ are benzyl, $R^4$ and $R^6$ are hydrogen atoms, and $-(CH_2)_nR^1$ is 3-oxopentyl,
$R^2$ is not a group selected from 6-benzyloxy-4-methoxy-3-methoxycarbonyl-2-pentylphenyl and 6-benzyloxy-3-benzyloxycarbonyl-4-methoxy-2-pentylphenyl,
(v) when $R^3$ is benzyl, $R^4$ is a hydrogen atom, $R^5$ is methyl, $-(CH_2)_nR^1$ is pentyl and $R^6$ is methoxycarbonyl or benzyloxycarbonyl,
$R^2$ is not 2,4-bis(benzyloxy)-6-(3-oxopentyl)phenyl,
(vi) when $R^3$ and $R^4$ are hydrogen atoms, $R^5$ is methyl, $-(CH_2)_nR^1$ is pentyl, and $R^6$ is carboxy or benzyloxycarbonyl,
$R^2$ is not 2,4-dihydroxy-6-(3-oxopentyl)phenyl, and
(vii) when $R^3$, $R^4$, and $R^6$ are hydrogen atoms, $R^5$ is n-propyl, and $-(CH_2)_nR^1$ is 5-(1,1-dimetylpropyl)-4-(2-hydrobenzotriazol-2-yl)-2-hydroxyphenylmethyl,
$R^2$ is not phenyl],
a prodrug thereof or a pharmaceutically acceptable salt thereof.

(2) The therapeutic agent for a tumor according to the above (1), wherein $R^2$ is a substituted or unsubstituted aromatic heterocyclic group, aryl substituted with 1-3 substituents or aryl.
(3) The therapeutic agent for a tumor according to the above (1), wherein $R^2$ is aryl substituted with 1-3 substituents or aryl.
(4) The therapeutic agent for a tumor according to the above (1), wherein $R^2$ is phenyl substituted with 1-3 substituents or phenyl.
(5) The therapeutic agent for a tumor according to the above (1), wherein $R^2$ is a substituted or unsubstituted aromatic heterocyclic group.
(6) The therapeutic agent for a tumor according to any of the above (1) to (5), wherein $R^3$ and $R^5$ may be the same or different, and each is a hydrogen atom, substituted or unsubstituted lower alkyl, substituted or unsubstituted lower alkanoyl, substituted or unsubstituted aroyl or substituted or unsubstituted lower alkenyl.
(7) The therapeutic agent for a tumor according to any of the above (1) to (5), wherein $R^3$, $R^4$, and $R^5$ each are hydrogen atoms.
(8) The therapeutic agent for a tumor according to any of the above (1) to (7), wherein $R^1$ is $CONR^7R^8$ (wherein $R^7$

and $R^8$ have the same meanings as defined above, respectively).

(9) The therapeutic agent for a tumor according to any of the above (1) to (7), wherein $R^1$ is $CONR^{7A}R^{8B}$ (wherein $R^{7A}$ and $R^{8A}$ may be the same or different, and each represents a hydrogen atom, substituted or unsubstituted lower alkyl, or substituted or unsubstituted heterocyclic alkyl).

(10) The therapeutic agent for a tumor according to any of the above (1) to (7), wherein $R^1$ is $CONR^{7B}R^{8B}$ (wherein $R^{7B}$ and $R^{8B}$ are combined together with the adjacent nitrogen atom thereto to form a substituted or unsubstituted heterocyclic group).

(11) The therapeutic agent for a tumor according to any of the above (1) to (7), wherein $R^1$ is substituted or unsubstituted lower alkoxy.

(12) The therapeutic agent for a tumor according to any of the above (1) to (11), wherein $R^6$ is a hydrogen atom, lower alkyl, halogen or aryl.

(13) The therapeutic agent for a tumor according to any of the above (1) to (11), wherein $R^6$ is lower alkyl.

(14) The therapeutic agent for a tumor according to any of the above (1) to (11), wherein $R^6$ is ethyl.

(15) The therapeutic agent for a tumor according to any of the above (1) to (14), wherein the tumor is a hematopoietic tumor.

(16) The therapeutic agent for a tumor according to the above (15), wherein the hematopoietic tumor is a tumor selected from leukemia, multiple myeloma and lymphoma.

(17) The therapeutic agent for a tumor according to any of the above (1) to (14), wherein the tumor is a solid tumor.

(18) The therapeutic agent for a tumor according to the above (17), wherein the solid tumor is a tumor selected from colon cancer, esophageal cancer, gastric cancer, hepatic cancer, pancreatic cancer, biliary tract cancer, bladder cancer, renal cancer, prostatic cancer, mammary cancer, uterine cervix cancer, uterine body cancer, ovarian cancer, head and neck cancer, lung cancer, osteosarcoma, melanoma, and brain tumor.

(19) A method for treating a tumor selected from a hematopoietic tumor and a solid tumor, comprising administering an effective amount of a benzoyl compound represented by General Formula (I) described in the above (1), a prodrug thereof or a pharmaceutically acceptable salt thereof.

(20) Use of a benzoyl compound represented by General Formula (I) described in the above (1), a prodrug thereof or a pharmaceutically acceptable salt thereof for the manufacture of a therapeutic agent for a tumor selected from a hematopoietic tumor and a solid tumor.

(21) A therapeutic agent for a tumor selected from a hematopoietic tumor and a solid tumor comprising, as an active ingredient, a benzoyl compound represented by General Formula (IA):

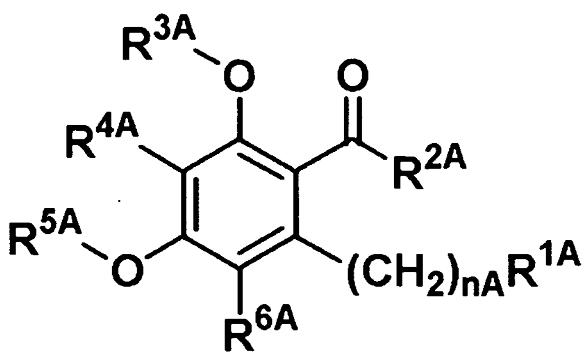

(IA)

[wherein

nA represents an integer of 0 to 10;

$R^{1A}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocyclic alkyl, substituted or unsubstituted aryl, substituted or unsubstituted arylsulfonyl, a substituted or unsubstituted heterocyclic group, $CONR^7R^8$ (wherein $R^7$ and $R^8$ may be the same or different, and each represents a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocyclic alkyl, or substituted or unsubstituted aroyl, or $R^7$ and $R^8$ are combined together with the adjacent nitrogen atom thereto to form a substituted or unsubstituted heterocyclic group), or $NR^9R^{10}$ (wherein $R^9$ and $R^{10}$ have the same meanings as the above $R^7$ and $R^8$, respectively);
$R^{2A}$ represents substituted or unsubstituted lower alkyl, substituted or unsubstituted lower alkenyl, substituted or unsubstituted lower alkynyl, substituted or unsubstituted cycloalkyl, substituted or unsubstituted aryl or a substituted or unsubstituted heterocyclic group;
$R^3$ and $R^{5A}$ may be the same or different, and each represents a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl; and
$R^{4A}$ and $R^{6A}$ may be the same or different, and each represents a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di (lower alkyl)amino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocyclic alkyl)],
a prodrug thereof, or a pharmaceutically acceptable salt thereof.

Effect of the Invention

The present invention provides a therapeutic agent for a tumor selected from a hematopoietic tumor and a solid tumor comprising, as an active ingredient, a benzoyl compound, a prodrug thereof, or a pharmaceutically acceptable salt thereof.

Brief Description of the Drawings

Fig. 1 shows the antitumor effect of Compound 33 on mice transplanted with human chronic myelocytic leukemia K562 cells. The abscissa axis represents the number of days after the start of the administration test, and the ordinate axis represents the tumor volume ($mm^3$). The results are expressed by the average values and standard deviations of five mice for each group.
Fig. 2 shows the antitumor effect of Compound 33 on mice transplanted with human lung cancer NCI-H596 cells. The abscissa axis represents the number of days after the start of the administration test, and the ordinate axis represents the tumor volume ($mm^3$). The results are expressed by the average values and standard deviations of five mice for each group.
Fig. 3 shows the antitumor effect of Compound 33 on mice transplanted with human prostate cancer 22Rv1 cells. The abscissa axis represents the number of days after the start of the administration test, and the ordinate axis represents the tumor volume ($mm^3$). The results are expressed by the average values and standard deviations of five mice for each group.

Reference Symbols

- • - : Group administered with Compound 33
- ○ - : Group not administered with drug

Best Mode for Carrying out the Invention

In the definitions of each groups in General Formula (I) and (IA):

Examples of the lower alkyl and lower alkyl moiety of the lower alkoxy, lower alkoxycarbonyl, lower alkylamino and di(lower alkyl)amino include straight-chain or branched alkyl groups having 1 to 8 carbon atoms, such as methyl, ethyl, propyl, isopropyl, butyl, sec-butyl, tert-butyl, pentyl, isopentyl, neopentyl, hexyl, heptyl and octyl. The two lower alkyl moieties of the di-lower alkylamino may be the same or different.
Examples of the lower alkenyl include straight-chain or branched alkenyl groups having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl groups having 2 to 8 carbon atoms, such as ethynyl, propynyl, butynyl, pentynyl, hexynyl, heptynyl and octynyl.
Examples of the lower alkanoyl and lower alkanoyl moiety of the lower alkanoyloxy include straight-chain or branched alkanoyl groups having 1 to 7 carbon atoms, such as formyl, acetyl, propionyl, butyryl, isobutyryl, valeryl, isovaleryl, pivaloyl, hexanoyl and heptanoyl.
Examples of the cycloalkyl include cycloalkyl groups having 3 to 8 carbon atoms, such as cyclopropyl, cyclobutyl, cyclopentyl, cyclohexyl, cycloheptyl and cyclooctyl.
Examples of the aryl and aryl moiety of the arylsulfonyl, aryloxy and aroyl include monocyclic, bicyclic or tricyclic aryl groups having 6 to 14 carbon atoms, such as phenyl, indenyl, naphthyl and anthryl.
Examples of the aralkyl include aralkyl groups having 7 to 15 carbon atoms, such as benzyl, phenethyl, benzhydryl and naphthylmethyl.
Examples of the aromatic heterocyclic group include 5- or 6-membered monocyclic aromatic heterocyclic groups containing at least one atom selected from a nitrogen atom, an oxygen atom and a sulfur atom, and bicyclic or tricyclic condensed-ring aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl, purinyl and benzodioxolanyl.
Examples of the heterocyclic group and heterocyclic moiety of the heterocyclic alkyl include groups described in the above definition of the aromatic heterocyclic groups and also alicyclic heterocyclic groups. Examples of the alicyclic heterocyclic group include 5- or 6-membered monocyclic alicyclic heterocyclic groups containing at least one atom selected from a nitrogen atom, an oxygen atom and a sulfur atom, and bicyclic or tricyclic condensed-ring alicyclic heterocyclic groups containing at least one atom selected from a nitrogen atom, an oxygen atom and a sulfur atom in which 3- to 8-membered rings are condensed, such as pyrrolidinyl, piperidino, piperidyl, piperazinyl, morpholino, morpholinyl, thiomorpholino, thiomorpholinyl, homopiperidino, homopiperazinyl, tetrahydropyridinyl, tetrahydroquinolinyl, tetrahydroisoquinolinyl, tetrahydrofuranyl, tetrahydropyranyl, dihydrobenzofuranyl, oxopiperazinyl and 2-oxopyrrolidinyl.
Examples of the heterocyclic group formed together with the adjacent nitrogen atom include 5- or 6-membered monocyclic heterocyclic groups containing at least one nitrogen atom (the monocyclic heterocyclic groups may also contain another nitrogen atom, an oxygen atom or a sulfur atom), and bicyclic or tricyclic condensed-ring heterocyclic groups containing at least one nitrogen atom in which 3- to 8-membered rings are condensed (the condensed-ring heterocyclic groups may also contain another nitrogen atom, an oxygen atom or a sulfur atom), such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, oxopiperazinyl and 2-oxopyrrolidinyl.

The alkylene moiety of the heterocyclic alkyl has the same meaning as a group produced by removing one hydrogen atom from the above-described lower alkyl.

The halogen means each atoms of fluorine, chlorine, bromine and iodine.

Examples of the substituents (A) in the substituted lower alkyl, the substituted lower alkoxy, the substituted lower alkoxycarbonyl, the substituted lower alkenyl and the substituted lower alkynyl include 1 to 3 substituents which may be the same or different, such as hydroxy, oxo, cyano, nitro, carboxy, amino, halogen, substituted or unsubstituted lower alkoxy, cycloalkyl, lower alkanoyl, lower alkoxycarbonyl, lower alkylamino and di(lower alkyl)amino. The position (s) to be substituted by the substituent(s) is/are not particularly limited. The halogen, the lower alkoxy, the cycloalkyl, the lower alkanoyl, the lower alkoxycarbonyl, the lower alkylamino and the di(lower alkyl)amino described as examples of substituents (A) each have the same meanings as defined above. Examples of the substituents in the substituted lower alkoxy described as an example of substituent (A) include 1 to 3 substituents which may be the same or different, such as hydroxy and halogen, and the halogen has the same meaning as defined above. Among the examples of substituents (A), preferred substituents in the substituted lower alkyl in the definitions of $R^7$ and $R^8$ described above include 1 to 3 substituents which may be the same or different, such as hydroxy or lower alkoxy.

Examples of substituents (B) in the substituted lower alkanoyl, the substituted lower alkanoyloxy, the substituted cycloalkyl, the substituted aryl, the substituted phenyl, the substituted arylsulfonyl, the substituted aryloxy, the substituted aralkyl, the substituted aroyl, the substituted heterocyclic alkyl, the substituted heterocyclic group, the substituted aromatic heterocyclic group and the substituted heterocyclic group formed together with the adjacent nitrogen atom include 1 to 3 substituents which may be the same or different, such as hydroxy, halogen, nitro, cyano, amino, carboxy, carbamoyl, substituted or unsubstituted lower alkyl, substituted or unsubstituted lower alkoxy, aralkyloxy, lower alkylsulfonyl, lower alkylsulfanyl, cycloalkyl, lower alkoxycarbonyl, lower alkylamino, di(lower alkyl)amino, lower alkanoyl, a heterocyclic group, substituted or unsubstituted aryl, substituted or unsubstituted heterocyclic alkyloxy, and substituted or unsubstituted heterocyclic carbonylalkyloxy. The position(s) to be substituted by substituent(s) is/are not particularly limited. The

halogen, the lower alkyl, the lower alkoxy, the cycloalkyl, the lower alkoxycarbonyl, the lower alkylamino, the di(lower alkyl)amino, the lower alkanoyl, the heterocyclic group and the aryl described as examples of substituents (B) each have the same meanings as defined above, the lower alkyl moiety of the lower alkylsulfonyl and lower alkylsulfanyl has the same meaning as the above-described lower alkyl, the aralkyl moiety of the aralkyloxy has the same meaning as the above-described aralkyl, and the heterocyclic group moiety and the alkylene of the heterocyclic alkyloxy and heterocyclic carbonylalkyloxy have the same meanings as the above-described heterocyclic group and the group produced by removing a hydrogen atom from the above-described lower alkyl, respectively. Examples of the substituents in the substituted lower alkyl, the substituted lower alkoxy and the substituted aryl described as examples of substituents (B) include 1 to 3 substituents which may be the same or different, such as hydroxy, halogen, lower alkoxy, cyano, lower alkylamino and di(lower alkyl)amino. Herein, the halogen, the lower alkoxy, the lower alkylamino and the di(lower alkyl) amino each have the same meanings as defined above. Examples of the substituents in the substituted heterocyclic alkyloxy and the substituted heterocyclic carbonylalkyloxy described as examples of substituents (B) include 1 to 3 substituents which may be the same or different, such as hydroxy, halogen, lower alkyl, lower alkoxy and a heterocyclic group. Herein, the halogen, the lower alkyl, the lower alkoxy and the heterocyclic group each have the same meanings as defined above. Among the examples of substituents (B), preferred substituents in the substituted aryl or substituted phenyl in the definition of $R^2$ described above include 1 to 3 substituents which may be the same or different, such as halogen, lower alkoxy, lower alkoxy-lower alkoxy, or substituted or unsubstituted heterocyclic alkyloxy. Further, as the substituent position in the substituted phenyl in the definition of $R^2$ described above, 3- and 4-position of phenyl or 3-position of phenyl are especially preferred.

Hereinafter, the compounds represented by General Formula (I) or (IA) are referred to as Compound (I) or (IA), respectively and the same applies to compounds of other formula numbers.

Compound (I) or (IA) used for the therapeutic agent for tumors of the present invention can be obtained according to the methods described in W02005/000778 or a similar method thereto.

Examples of Compound (I) or (IA) used for the therapeutic agent for tumors of the present invention are shown in Table 1 and Table 2.

In the tables, Ph represents phenyl, and the numbers preceding the groups in $R^{2a}$, $R^{2b}$ and $R^{2c}$ refer to the substituted positions in phenyl.

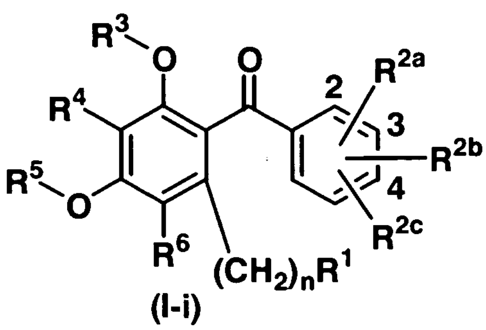

(I-i)

[Table 1-1]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 1 | $OCH_3$ | 2 | H | H | H | H | H | H | H |
| 2 | $OCH_3$ | 2 | H | H | H | H | H | H | Br |
| 3 | $OCH_3$ | 2 | H | H | H | H | H | H | Ph |
| 4 | $OCH_3$ | 2 | H | H | H | H | H | H | $COCH_3$ |
| 5 | $CO_2CH_3$ | 1 | H | H | H | H | H | H | $CH_2CH_3$ |
| 6 | $CO_2CH_3$ | 1 | 3-$OCH_3$ | H | H | H | H | H | $CH_2CH_3$ |
| 7 | $OCH_3$ | 2 | H | H | H | H | H | H | $CH_2CH_3$ |
| 8 | $CO_2CH_3$ | 1 | 4-$OCH_3$ | H | H | H | H | H | $CH_2CH_3$ |
| 9 | $OCH_3$ | 2 | 4-$OCH_3$ | H | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 10 | CON(CH3)$CH_2CH_2OCH_3$ | 1 | 4-$OCH_3$ | H | H | H | H | H | $CH_2CH_3$ |
| 11 | $OCH_3$ | 2 | 4-$NO_2$ | H | H | H | H | H | $CH_2CH_3$ |
| 12 | $OCH_2CH_2OCH_3$ | 2 | 4-$OCH_3$ | H | H | H | H | H | $CH_2CH_3$ |
| 13 | $CON(CH_2CH_2OH)_2$ | 1 | 4-$OCH_3$ | H | H | H | H | H | $CH_2CH_3$ |
| 14 | $CON(CH_3)CH_2CH_2OH$ | 1 | 4-$OCH_3$ | H | H | H | H | H | $CH_2CH_3$ |
| 15 | $CO_2CH_3$ | 1 | 4-$OCH_3$ | H | H | H | H | H | I |
| 16 | 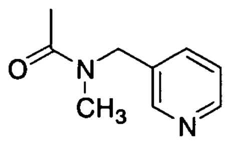 | 1 | 4-$OCH_3$ | H | H | H | H | H | $CH_2CH_3$ |

[Table 1-2]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 17 | 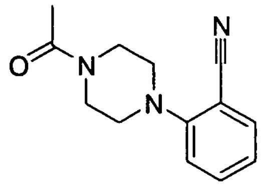 | 1 | 4-$OCH_3$ | H | H | H | H | H | $CH_2CH_3$ |
| 18 | $CO_2CH_3$ | 1 | 4-$OCH_3$ | H | H | H | H | $CH_2CH=CH_2$ | H |
| 19 | $CO_2CH_3$ | 1 | 4-$OCH_3$ | H | H | H | H | H | H |
| 20 | $CO_2CH_3$ | 1 | 4-OH | H | H | H | H | H | H |
| 21 | 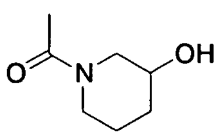 | 1 | 4-$OCH_3$ | H | H | H | H | H | $CH_2CH_3$ |
| 22 | 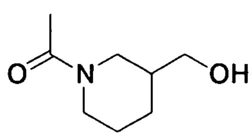 | 1 | 4-$OCH_3$ | H | H | H | H | H | $CH_2CH_3$ |
| 23 | 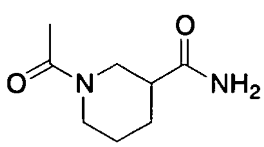 | 1 | 4-$OCH_3$ | H | H | H | H | H | $CH_2CH_3$ |
| 24 | 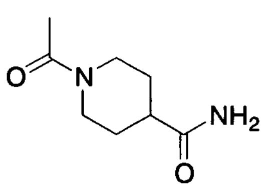 | 1 | 4-$OCH_3$ | H | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 25 | 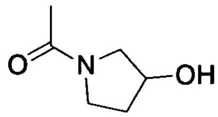 | 1 | 4-$OCH_3$ | H | H | H | H | H | $CH_2CH_3$ |
| 26 | CON $(CH_3)$ $CH_2CH$ (OH) $CH_2OH$ | 1 | 4-$OCH_3$ | H | H | H | H | H | $CH_2CH_3$ |
| 27 | $CO_2CH_3$ | 1 | 4-$OCH_3$ | H | H | $CH_3$ | H | H | H |

[Table 1-3]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 28 | 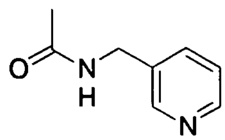 | 1 | 4-$OCH_3$ | H | H | H | H | H | $CH_2CH_3$ |
| 29 | $CO_2CH_3$ | 1 | 3-$OCH_3$ | 4-$OCH_3$ | H | H | H | H | $CH_2CH_3$ |
| 30 | 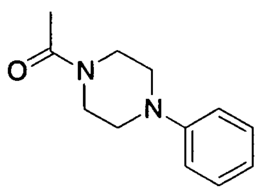 | 1 | 4-$OCH_3$ | H | H | H | H | H | $CH_2CH_3$ |
| 31 | 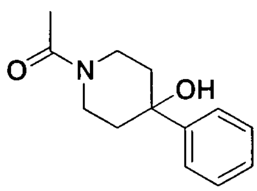 | 1 | 4-$OCH_3$ | H | H | H | H | H | $CH_2CH_3$ |
| 32 | 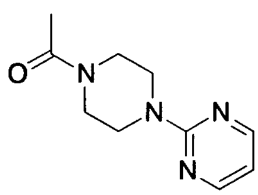 | 1 | 4-$OCH_3$ | H | H | H | H | H | $CH_2CH_3$ |
| 33 | $CON(CH_2CH_2OH)CH_2CH_2OCH_3$ | 1 | 4-$OCH_3$ | H | H | H | H | H | $CH_2CH_3$ |
| 34 | $OCH_2CH(OH)CH_2OH$ | 2 | 2-F | 4-$OCH_3$ | H | H | H | H | $CH_2CH_3$ |
| 35 | 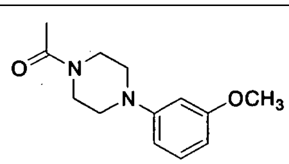 | 1 | 4-$OCH_3$ | H | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 36 | 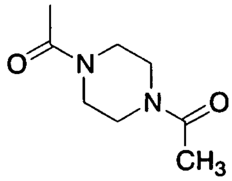 | 1 | 4-$OCH_3$ | H | H | H | H | H | $CH_2CH_3$ |

[Table 1-4]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 37 | 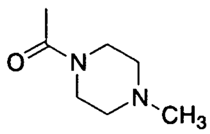 | 1 | 4-$OCH_3$ | H | H | H | H | H | $CH_2CH_3$ |
| 38 | 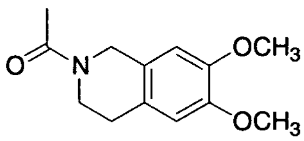 | 1 | 4-$OCH_3$ | H | H | H | H | H | $CH_2CH_3$ |
| 39 | 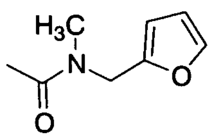 | 1 | 4-$OCH_3$ | H | H | H | H | H | $CH_2CH_3$ |
| 40 | 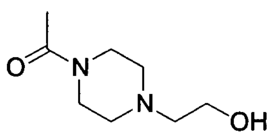 | 1 | 4-$OCH_3$ | H | H | H | H | H | $CH_2CH_3$ |
| 41 | 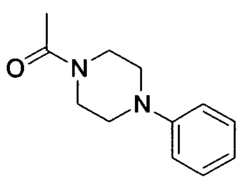 | 1 | 3-$OCH_3$ | 4-$OCH_3$ | H | H | H | H | $CH_2CH_3$ |
| 42 | $CON(CH_3)_2$ | 1 | 4-$OCH_3$ | H | H | H | H | H | $CH_2CH_3$ |
| 43 | 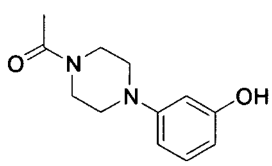 | 1 | 4-$OCH_3$ | H | H | H | H | H | $CH_2CH_3$ |
| 44 | 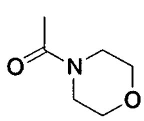 | 1 | 4-$OCH_3$ | H | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 45 | 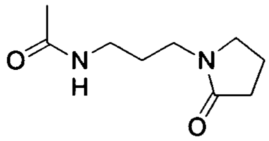 | 1 | 4-$OCH_3$ | H | H | H | H | H | $CH_2CH_3$ |

[Table 1-5]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 46 | $OCH_2CH(OH)CH_2OH$ | 2 | 4-$OCH_3$ | H | H | H | H | H | $CH_2CH_3$ |
| 47 | $CONHCH(CH_2OH)_2$ | 1 | 4-$OCH_3$ | H | H | H | H | H | $CH_2CH_3$ |
| 48 | $CONHCCH_3(CH_2OH)_2$ | 1 | 4-$OCH_3$ | H | H | H | H | H | $CH_2CH_3$ |
| 49 | $CON(CH_2CH_2OH)_2$ | 1 | 3-$OCH_3$ | 4-$OCH_3$ | H | H | H | H | $CH_2CH_3$ |
| 50 | $CON(CH_2CH_2OH)_2$ | 1 | 4-F | H | H | H | H | H | $CH_2CH_3$ |
| 51 | $OCH_2CH(OH)CH_2OH$ | 2 | 3-$OCH_3$ | 4-$OCH_3$ | H | H | H | H | $CH_2CH_3$ |
| 52 | $OCH_2CH(OH)CH_2OH$ | 2 | 3-F | 4-$OCH_3$ | H | H | H | H | $CH_2CH_3$ |
| 53 | $OCH_2CH(OH)CH_2OH$ | 2 | 3-$OCH_3$ | 4-$OCH_3$ | 5-$OCH_3$ | H | H | H | $CH_2CH_3$ |
| 54 | 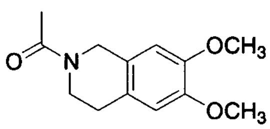 | 1 | 4-F | H | H | H | H | H | $CH_2CH_3$ |
| 55 | 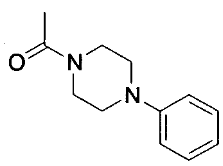 | 1 | 4-OH | H | H | H | H | H | $CH_2CH_3$ |
| 56 | $CON(CH_2CH_2OH)$ $CH_2CH_2OCH_3$ | 1 | 3-$OCH_3$ | 4-$OCH_3$ | H | H | H | H | $CH_2CH_3$ |
| 57 | $OCH_2CH(OH)CH_2OH$ | 2 | 3-Cl | 4-F | H | H | H | H | $CH_2CH_3$ |
| 58 | $OCH_2CH(OH)CH_2OH$ | 2 | 3 - 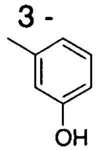 | 4-$OCH_3$ | H | H | H | H | $CH_2CH_3$ |
| 59 | $OCH_2CH(OH)CH_2OH$ | 2 | 3 - 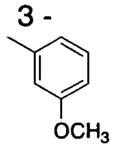 | 4-$OCH_3$ | H | H | H | H | $CH_2CH_3$ |

[Table 1-6]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 60 | $OCH_2CH_2OH$ | 2 | 4-$OCH_3$ | H | H | H | H | H | $CH_2CH_3$ |
| 61 | 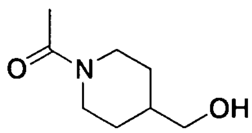 | 1 | 4-$OCH_3$ | H | H | H | H | H | $CH_2CH_3$ |
| 62 | $OCH_2CH_2OH$ | 2 | H | H | H | H | H | H | $CH_2CH_3$ |
| 63 | $OCH_2CH_2OH$ | 2 | 3-OH | 4-$OCH_3$ | H | H | H | H | $CH_2CH_3$ |
| 64 | 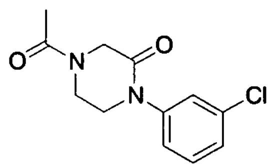 | 1 | 4-$OCH_3$ | H | H | H | H | H | $CH_2CH_3$ |
| 65 | $OCH_2CH_2OH$ | 2 | 4-$OCHF_2$ | H | H | H | H | H | $CH_2CH_3$ |
| 66 | $CON(CH_2CH_2OH)CH_2CH_2OCH_3$ | 1 | 4-F | H | H | H | H | H | $CH_2CH_3$ |
| 67 | 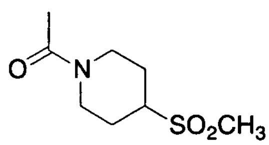 | 1 | 4-$OCH_3$ | H | H | H | H | H | $CH_2CH_3$ |
| 68 | 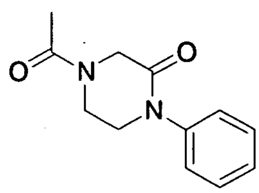 | 1 | 4-$OCH_3$ | H | H | H | H | H | $CH_2CH_3$ |
| 69 | 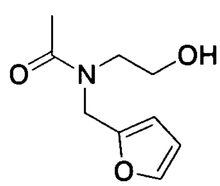 | 1 | 4-$OCH_3$ | H | H | H | H | H | $CH_2CH_3$ |

[Table 1-7]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 75 | 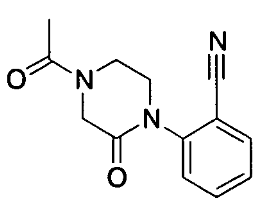 | 1 | H | H | H | H | H | H | $CH_2CH_3$ |
| 76 | $CON(CH_2CH_2OH)_2$ | 1 | H | H | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 77 | $CON(CH_2CH_2OH)CH_2CH_2OCH_3$ | 1 | H | H | H | H | H | H | $CH_2CH_3$ |
| 78 | 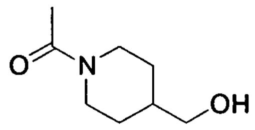 | 1 | H | H | H | H | H | H | $CH_2CH_3$ |
| 79 | $CON(CH_2CH_2OH)CH_2CH_2OCH_3$ | 1 | 3-OH | H | H | H | H | H | $CH_2CH_3$ |
| 80 | $CON(CH_2CH_2OH)_2$ | 1 | 4-OH | H | H | H | H | H | $CH_2CH_3$ |
| 81 | $CON(CH_2CH_2OH)CH_2CH_2OCH_3$ | 1 | 4-OH | H | H | H | H | H | $CH_2CH_3$ |
| 82 | 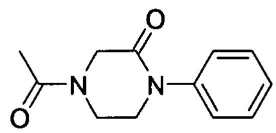 | 1 | 4-F | H | H | H | H | H | $CH_2CH_3$ |
| 83 | $CON(CH_2CH_2OH)_2$ | 1 | 3-OH | 4-$OCH_3$ | H | H | H | H | $CH_2CH_3$ |
| 84 | $CON(CH_2CH_2OH)CH_2CH_2OCH_3$ | 1 | 3-OH | 4-$OCH_3$ | H | H | H | H | $CH_2CH_3$ |
| 85 | $CON(CH_2CH_2OH)_2$ | 1 | 3-F | 4-$OCH_3$ | H | H | H | H | $CH_2CH_3$ |
| 86 | $CON(CH_2CH_2OH)CH_2CH_2OCH_3$ | 1 | 3-F | 4-$OCH_3$ | H | H | H | H | $CH_2CH_3$ |
| 87 | $CON(CH_2CH_2OH)_2$ | 1 | 4-$OCF_3$ | H | H | H | H | H | $CH_2CH_3$ |
| 88 | $CON(CH_2CH_2OH)CH_2CH_2OCH_3$ | 1 | 4-$OCF_3$ | H | H | H | H | H | $CH_2CH_3$ |
| 89 | $CON(CH_2CH_2OCH_3)_2$ | 1 | 3-OH | 4-$OCH_3$ | H | H | H | H | $CH_2CH_3$ |

[Table 1-8]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 90 | $CON(CH_2CH_2OH)_2$ | 1 | 4-$OCHF_2$ | H | H | H | H | H | $CH_2CH_3$ |
| 91 | $CON(CH_2CH_2OH)CH_2CH_2OCH_3$ | 1 | 4-$OCHF_2$ | H | H | H | H | H | $CH_2CH_3$ |
| 92 | $CON(CH_2CH_2OH)_2$ | 1 | 3-OH | 4-$CH_3$ | H | H | H | H | $CH_2CH_3$ |
| 93 | $CON(CH_2CH_2OH)CH_2CH_2OCH_3$ | 1 | 3-OH | 4-$CH_3$ | H | H | H | H | $CH_2CH_3$ |
| 94 | $CON(CH_2CH_2OH)CH_2CH_2CH_2OH$ | 1 | 4-$OCF_3$ | H | H | H | H | H | $CH_2CH_3$ |
| 95 | $CON(CH_2CH_2OH)CH_2CH_2OCH_3$ | 1 | 4-$SCH_3$ | H | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 96 | CON $(CH_2CH_2OH)_2$ | 1 | 4-$SO_2CH_3$ | H | H | H | H | H | $CH_2CH_3$ |
| 97 | CON$(CH_2CH_2OH)$ $CH_2CH_2OCH_3$ | 1 | 4-$SO_2CH_3$ | H | H | H | H | H | $CH_2CH_3$ |
| 98 | 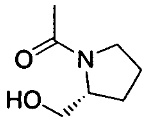 | 1 | 4-$OCH_3$ | H | H | H | H | H | $CH_2CH_3$ |
| 99 | CON$(CH_2CH_2OH)$ $CH_2CH_2CH_2OH$ | 1 | 3 -$OCH_3$ | 4-$OCH_3$ | H | H | H | H | $CH_2CH_3$ |
| 100 | 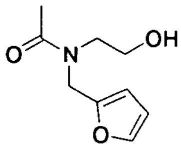 | 1 | 3-$OCH_3$ | 4-$OCH_3$ | H | H | H | H | $CH_2CH_3$ |
| 101 | CON$(CH_2CH_2OH)$ $CH_2CH_2CH_2OH$ | 1 | 4-$OCH_3$ | H | H | H | H | H | $CH_2CH_3$ |
| 102 | CON$(CH_2CH_2OH)$ $CH_2CH_2OCH_3$ | 1 | 3-$OCH_2CH_2$-OH | 4-$OCH_3$ | H | H | H | H | $CH_2CH_3$ |
| 103 | CON$(CH_2CH_2OH)$ $CH_2CH_2OCH_3$ | 1 | 3- $OCH_2CH_2$-$OCH_3$ | 4-$OCH_3$ | H | H | H | H | $CH_2CH_3$ |
| 104 | CON$(CH_2CH_2OH)$ $CH_2CH_2OCH_3$ | 1 | 3- 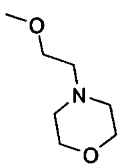 | 4-$OCH_3$ | H | H | H | H | $CH_2CH_3$ |

[Table 1-9]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 105 | $CON(CH_2CH_2OH)CH_2CH_2OCH_3$ | 1 | 3-$OCH_3$ | 4-OH | H | H | H | H | $CH_2CH_3$ |
| 106 | $CON(CH_2CH_2OCH_3)_2$ | 1 | 3-$OCH_3$ | 4-OH | H | H | H | H | $CH_2CH_3$ |
| 107 | $CON(CH_2CH_2OCH_3)_2$ | 1 | 4-$SO_2CH_3$ | H | H | H | H | H | $CH_2CH_3$ |
| 108 | $CON(CH_2CH_2OH)CH_2CH_2OCH_3$ | 1 | 3-$OCH_3$ | 4-$OCH_2$-$CH_2OH$ | H | H | H | H | $CH_2CH_3$ |
| 109 | $CON(CH_2CH_2OH)CH_2CH_2OCH_3$ | 1 | 3-$OCH_3$ | 4-$OCH_2$-$CH_2OCH_3$ | H | H | H | H | $CH_2CH_3$ |
| 110 | $CON(CH_2CH_2OH)CH_2CH_2OCH_3$ | 1 | 3-$OCH_3$ | 4 – 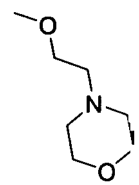 | H | H | H | H | $CH_2CH_3$ |
| 111 | 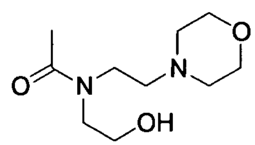 | 1 | 4-$OCH_3$ | H | H | H | H | H | $CH_2CH_3$ |
| 112 | $CON(CH_2CH_2OCH)CH_2CH_2N(CH_3)_2$ | 1 | 3-$OCH_3$ | 4-$OCH_3$ | H | H | H | H | $CH_2CH_3$ |
| 113 | $CON(CH_2CH_2OCH3)CH_2CH_2N(CH_3)_2$ | 1 | 4-$OCH_3$ | H | H | H | H | H | $CH_2CH_3$ |
| 114 | $CON(CH_2CH_2OH)$<br>$CH_2CH_2N$-$(CH_2CH_3)_2$ | 1 | 3-$OCH_3$ | 4-$OCH_3$ | H | H | H | H | $CH_2CH_3$ |
| 115 | 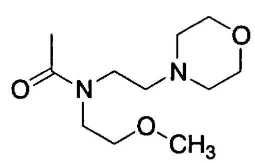 | 1 | 3-$OCH_3$ | 4-$OCH_3$ | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 116 | 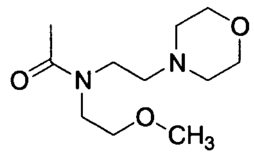 | 1 | 4-$OCH_3$ | H | H | H | H | H | $CH_2CH_3$ |

[Table 1-10]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 117 | CON ($(CH_2CH_2CH_2OH)$) $CH_2CH_2$-$OCH_3$ | 1 | 4-$OCH_3$ | H | H | H | H | H | $CH_2CH_3$ |
| 118 | CON($CH_2CH_2OH$) $CH_2CH_2CH_2$-$OCH_3$ | 1 | 4-$OCH_3$ | H | H | H | H | H | $CH_2CH_3$ |
| 119 | CON ($(CH_2CH_2CH_2OH)$) $CH_2CH_2$-$OCH_3$ | 1 | 3-$OCH_3$ | 4-$OCH_3$ | H | H | H | H | $CH_2CH_3$ |
| 120 | CON($CH_2CH_2OH$) $CH_2CH_2CH_2$-$OCH_3$ | 1 | 3-$OCH_3$ | 4-$OCH_3$ | H | H | H | H | $CH_2CH_3$ |
| 121 | CON$(CH_2CH_2OH)_2$ | 1 | 4-$OCH_2CH_3$ | H | H | H | H | H | $CH_2CH_3$ |
| 122 | CON($CH_2CH_2OH$) $CH_2CH_2OCH_3$ | 1 | 4-$OCH_2CH_3$ | H | H | H | H | H | $CH_2CH_3$ |
| 123 | CON$(CH_2CH_2OH)_2$ | 1 | 4-OCH $(CH_3)_2$ | H | H | H | H | H | $CH_2CH_3$ |
| 124 | CON($CH_2CH_2OH$) $CH_2CH_2OCH_3$ | 1 | 4-OCH $(CH_3)_2$ | H | H | H | H | H | $CH_2CH_3$ |
| 125 | CON $(CH_2CH_2OCH_3)_2$ | 1 | 3-$OCH_3$ | 4- 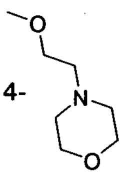 | H | H | H | H | $CH_2CH_3$ |
| 126 | CON $(CH_2CH_2OCH_3)_2$ | 1 | 3-$OCH_3$ | 4-$OCH_2$-$CH_2OH$ | H | H | H | H | $CH_2CH_3$ |
| 127 | CON ($CH_2CH_2OCH_3$) $CH_2CH_2$-$CH_2N$ $(CH_3)_2$ | 1 | 3-$OCH_3$ | 4-$OCH_3$ | H | H | H | H | $CH_2CH_3$ |
| 128 | CON ($CH_2CH_2OCH_3$) $CH_2CH_2$-$CH_2N$ $(CH_3)_2$ | 1 | 4-$OCH_3$ | H | H | H | H | H | $CH_2CH_3$ |
| 129 | CON ($CH_2CH_2OCH_3$) $CH_2CH_2$-N $(CH_2CH_3)_2$ | 1 | 3-$OCH_3$ | 4-$OCH_3$ | H | H | H | H | $CH_2CH_3$ |
| 130 | CON ($CH_2CH_2OCH_3$) $CH_2CH_2$-N $(CH_2CH_3)_2$ | 1 | 4-$OCH_3$ | H | H | H | H | H | $CH_2CH_3$ |

[Table 1-11]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 131 | 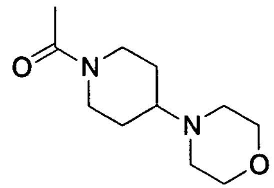 | 1 | 3-$OCH_3$ | 4-$OCH_3$ | H | H | H | H | $CH_2CH_3$ |
| 132 | 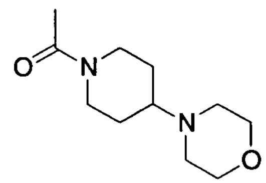 | 1 | 4-$OCH_3$ | H | H | H | H | H | $CH_2CH_3$ |
| 133 | 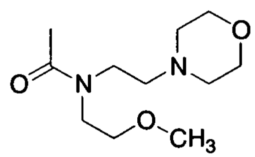 | 1 | 4-$OCH_2CH_3$ | H | H | H | H | H | $CH_2CH_3$ |
| 134 | 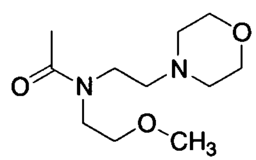 | 1 | 4-$OCH(CH_3)_2$ | H | H | H | H | H | $CH_2CH_3$ |
| 135 | $CON(CH_2CH_2OH)CH_2CH_2OCH$ | 1 | 4-$OCH_3$ | H | H | H | H | H | Br |
| 136 | $CON(CH_2CH_2OH)CH_2CH_2OCH$ | 1 | 4-$OCH_3$ | H | H | H | H | H | $COCH_3$ |
| 137 | $CON(CH_2CH_2OH)_2$ | 1 | 3-$OCH_2$-$CH_2OCH_3$ | 4-$OCH_2$-$CH_2OCH_3$ | H | H | H | H | $CH_2CH_3$ |
| 138 | $CON(CH_2CH_2OH)CH_2CH_2OCH$ | 1 | 3-$OCH_2$-$CH_2OCH_3$ | 4-$OCH_2$-$CH_2OCH_3$ | H | H | H | H | $CH_2CH_3$ |

(continued)

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 139 | $CON(CH_2CH_2OCH_3)_2$ | 1 | 3-$OCH_3$ | 4- 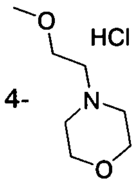 HCl | H | H | H | H | $CH_2CH_3$ |
| 140 | $CON(CH_2CH_2CH_2OCH_3)$ -$CH_2CH_2N(CH_3)_2$ | 1 | 4-$OCH_3$ | H | H | H | H | H | $CH_2CH_3$ |

[Table 1-12]

| Compound | $R^1$ | n | $R^{2a}$ | $R^{2b}$ | $R^{2c}$ | $R^3$ | $R^4$ | $R^5$ | $R^6$ |
|---|---|---|---|---|---|---|---|---|---|
| 141 | CON $(CH_2CH_2CH_2OCH_3)$ $CH_2$-$CH_2N(CH_3)_2$ | 1 | 3-$OCH_3$ | 4-$OCH_3$ | H | H | H | H | $CH_2CH_3$ |
| 142 | $CON(CH_2CH_2OCH_3)$ $CH_2CH_2$ -$N(CH_3)_2$·HCl | 1 | 4-$OCH_3$ | H | H | H | H | H | $CH_2CH_3$ |
| 143 | $CON(CH_2CH_2OH)_2$ | 1 | 4-$CF_3$ | H | H | H | H | H | $CH_2CH_3$ |
| 144 | $CON(CH_2CH_2OH)$ $CH_2CH_2OCH_3$ | 1 | 4-$CF_3$ | H | H | H | H | H | $CH_2CH_3$ |
| 145 | $CON(CH_2CH_2OH)_2$ | 1 | 3-F | 4-F | H | H | H | H | $CH_2CH_3$ |
| 146 | $CON(CH_2CH_2OH)$ $CH_2CH_2OCH_3$ | 1 | 3-F | 4-F | H | H | H | H | $CH_2CH_3$ |
| 147 | $CON(CH_2CH_2OCH_3)_2$ | 1 | 3-$OCH_3$ | | H | H | H | H | $CH_2CH_3$ |
| 148 | $CON(CH_2CH_2OCH_3)_2$ | 1 | 3-$OCH_3$ | 4- 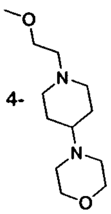 | H | H | H | H | $CH_2CH_3$ |
| 149 | $CON(CH_2CH_2OCH_3)_2$ | 1 | 3-$OCH_3$ | 4- 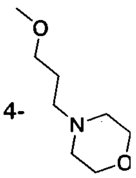 | H | H | H | H | $CH_2CH_3$ |
| 150 | $CON(CH_2CH_2OCH_3)_2$ | 1 | 3-$OCH_3$ | 4- 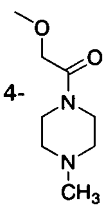 | H | H | H | H | $CH_2CH_3$ |

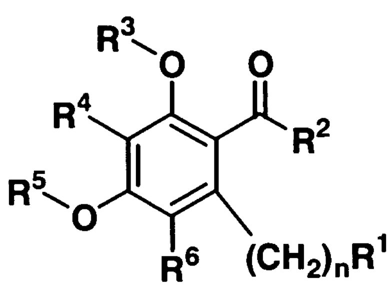

(I-ii)

[Table 2]

| Compound | $R^1$ | n | $R^2$ | $R^3$ | $R^4$ | $R^5$ | $R^6$ |
|---|---|---|---|---|---|---|---|
| 70 | $OCH_2CH(OH)CH_2OH$ | 2 | 4-pyridyl | H | H | H | $CH_2CH_3$ |
| 71 | $OCH_2CH_2OH$ | 2 | 3-thienyl | H | H | H | $CH_2CH_3$ |
| 72 | $OCH_2CH_2OH$ | 2 | 2-thienyl | H | H | H | $CH_2CH_3$ |
| 73 | $OCH_2CH_2OH$ | 2 | 3-furyl | H | H | H | $CH_2CH_3$ |
| 74 | $CON(CH_2CH_2OH)_2$ | 1 | 3-thienyl | H | H | H | $CH_2CH_3$ |
| 151 | $CON(CH_2CH_2OH)CH_2CH_2OCH$, | 1 | 3-thienyl | H | H | H | $CH_2CH_3$ |
| 152 | $CON(CH_2CH_2OH)_2$ | 1 | 3-furyl | H | H | H | $CH_2CH_3$ |
| 153 | $CON(CH_2CH_2OH)CH_2CH_2OCH_3$ | 1 | 3-furyl | H | H | H | $CH_2CH_3$ |
| 154 | 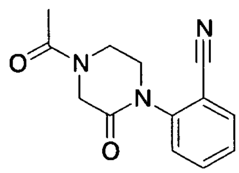 | 1 | 3-thienyl | H | H | H | $CH_2CH_3$ |
| 155 | 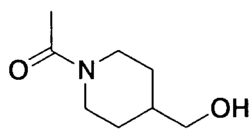 | 1 | 3-thienyl | H | H | H | $CH_2CH_3$ |
| 156 | $CON(CH_2CH_2OCH_3)_2$ | 1 | 3-furyl | H | H | H | $CH_2CH_3$ |
| 157 | $CON(CH_2CH_2OH)_2$ | 1 | 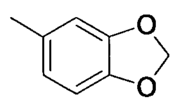 | H | H | H | $CH_2CH_3$ |
| 158 | $CON(CH_2CH_2OH)CH_2CH_2OCH_3$ | 1 | 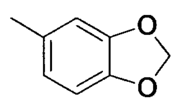 | H | H | H | $CH_2CH_3$ |

The prodrugs of Compound (I) or (IA) used for the therapeutic agent for tumors of the present invention include compounds which are converted in vivo, for example, by various mechanisms such as hydrolysis in blood to form Compound (I) or (IA) of the present invention. Such compounds can be specified by techniques well known in the art (e.g. J. Med. Chem., 1997, Vol. 40, p. 2011-2016; Drug Dev. Res., 1995, Vol. 34, p. 220-230; Advances in Drug Res., 1984, Vol. 13, p. 224-331; Bundgaard, Design of Prodrugs, 1985, Elsevier Press and the like).

Specifically, when Compound (I) or (IA) has carboxy in its structure, examples of prodrugs of Compound (I) or (IA) include compounds in which the hydrogen atom of said carboxy is substituted by a group selected from lower alkyl, lower alkanoyloxyalkyl [e.g. lower alkanoyloxymethyl, 1-(lower alkanoyloxy)ethyl and 1-methyl-1-(lower alkanoyloxy) ethyl], lower alkoxycarbonyloxyalkyl [e.g. lower alkoxycarbonyloxymethyl, 1-(lower alkoxycarbonyloxy)ethyl, and 1-methyl-1-(lower alkoxycarbonyloxy)ethyl], N-(lower alkoxycarbonyl)aminoalkyl {e.g. N-(lower alkoxycarbonyl)aminomethyl and 1-[N-(lower alkoxycarbonyl)amino]ethyl}, 3-phthalidyl, 4-crotonolactonyl, $\gamma$-butyrolacton-4-yl, di(lower alkyl)aminoalkyl, carbamoylalkyl, di(lower alkyl)carbamoylalkyl, piperidinoalkyl, pyrrolidinoalkyl, morpholinoalkyl and the like.

Also, when Compound (I) or (IA) has alcoholic hydroxy in its structure, examples of prodrugs of Compound (I) or (IA) include compounds in which the hydrogen atom of said hydroxy is substituted by a group selected from lower alkanoyloxyalkyl, 1-(lower alkanoyloxy)ethyl, 1-methyl-1-(lower alkanoyloxy)ethyl, lower alkoxycarbonyloxyalkyl, N-(lower alkoxycarbonyl)aminoalkyl, succinoyl, lower alkanoyl, $\alpha$-amino lower alkanoyl and the like.

Also, when Compound (I) or (IA) has amino in its structure, examples of prodrugs of Compound (I) or (IA) include compounds in which one or two hydrogen atoms of said amino are substituted by a group selected from lower alkylcarbonyl, lower alkoxycarbonyl, lower alkylcarbamoyl, di-lower alkylcarbamoyl and the like.

The lower alkyl and lower alkyl moiety of the above-described lower alkoxycarbonyloxyalkyl, lower alkoxycarbonyloxymethyl, 1-(lower alkoxycarbonyloxy)ethyl, 1-methyl-1-(lower alkoxycarbonyloxy)ethyl, N-(lower alkoxycarbonyl)aminoalkyl, N-(lower alkoxycarbonyl)aminomethyl, 1-[N-(lower alkoxycarbonyl)amino]ethyl, di(lower alkyl)aminoalkyl, di(lower alkyl)carbamoylalkyl, lower alkoxycarbonyloxymethyl, lower alkylcarbonyl, lower alkoxycarbonyl, lower alkylcarbamoyl and di(lower alkyl)carbamoyl has the same meaning as the above-described lower alkyl. The two lower alkyl moieties of the di(lower alkyl)aminoalkyl, di(lower alkyl)carbamoylalkyl and di(lower alkyl)carbamoyl may be the same or different.

Also, the lower alkanoyl moiety of the above-described lower alkanoyloxyalkyl, lower alkanoyloxymethyl, 1-(lower alkanoyloxy)ethyl, 1-methyl-1-(lower alkanoyloxy)ethyl, lower alkanoyl and $\alpha$-amino lower alkanoyl has the same meaning as the above-described lower alkanoyl.

Also, the alkylene moiety of the above-described lower alkanoyloxyalkyl, lower alkoxycarbonyloxyalkyl, N-(lower alkoxycarbonyl)aminoalkyl, di(lower alkyl)aminoalkyl, carbamoylalkyl, di(lower alkyl)carbamoylalkyl, piperidinoalkyl, pyrrolidinoalkyl and morpholinoalkyl has the same meaning as the group produced by removing a hydrogen atom from the above-described lower alkyl.

These prodrugs of Compound (I) or (IA) can be prepared from Compound (I) according to, for example, the methods described in T.W. Greene, Protective Groups in Organic Synthesis, third edition, John Wiley & Sons Inc. (1999), or methods similar thereto.

The pharmaceutically acceptable salts of Compound (I) or (IA), or prodrugs thereof include pharmaceutically acceptable acid addition salts, metal salts, ammonium salts, organic amine addition salts, amino acid addition salts, and the like.

Examples of the pharmaceutically acceptable acid addition salts of Compounds (I) or (IA), or prodrugs thereof include inorganic acid addition salts such as hydrochloride, sulfate, nitrate and phosphate, and organic acid addition salts such as acetate, maleate, fumarate and citrate. Examples of the pharmaceutically acceptable metal salts include alkali metal salts such as sodium salt and potassium salt, alkaline earth metal salts such as magnesium salt and calcium salt, aluminum salt, and zinc salt. Examples of the pharmaceutically acceptable ammonium salts include ammonium and tetramethylammonium. Examples of the pharmaceutically acceptable organic amine addition salts include an addition salt of morpholine or piperidine. Examples of the pharmaceutically acceptable amino acid addition salts include an addition salt of glycine, phenylalanine, lysine, aspartic acid, glutamic acid, or the like.

Examples of the hematopoietic tumors to be treated by the therapeutic agent for tumors of the present invention include leukemia such as acute myelocytic leukemia (AML), acute lymphocytic leukemia (ALL), acute promyelocytic leukemia (APL), chronic myelocytic leukemia (CML), chronic lymphocytic leukemia (CLL), hairy cell leukemia (HCL), and adult T-cell leukemia, Hodgkin's disease, lymphoma such as non-Hodgkin's lymphoma (for example, B-cell lymphoma, T-cell lymphoma and the like), multiple myeloma and the like.

Examples of the solid tumors to be treated by the therapeutic agent for a tumor of the present invention include digestive tumors such as colon cancer, esophageal cancer, gastric cancer, hepatic cancer, pancreatic cancer, biliary tract cancer, urinary cancer or tumors such as bladder cancer, renal cancer, prostatic cancer, gynecologic tumors such as mammary cancer, uterine cervix cancer, uterine body cancer, ovarian cancer, head and neck cancer, lung cancer, osteosarcoma, melanoma, brain tumor and the like.

Although Compound (I) or (IA), prodrugs thereof, or pharmaceutically acceptable salts thereof used for the therapeutic agent for tumors of the present invention can be administered as such, it is generally preferred to offer them in the form of various pharmaceutical preparations. Such pharmaceutical preparations are to be used in animals and humans.

The pharmaceutical preparations of the present invention can comprise Compound (I), (IA), prodrugs thereof, or pharmaceutically acceptable salts thereof as the active ingredient alone or in combination with any other active ingredients for the therapy. These pharmaceutical preparations may be produced by any methods well known in the technical field of pharmaceutics by mixing the active ingredient with one or more pharmaceutically acceptable carriers.

It is desirable to select a route of administration that is most effective for the therapy, examples thereof being oral administration or parenteral administration such as intravenous administration.

Examples of the dosage form include tablets, injections, and the like.

Preparations suitable for oral administration such as tablets can be produced using, for example, excipients (e.g., lactose and mannitol), disintegrators (e.g., starch), lubricants (e.g., magnesium stearate), binders (e.g., hydroxypropyl cellulose), surfactants (e.g., fatty acid esters) and plasticizers (e.g., glycerin).

Preparations suitable for parenteral administration preferably comprise a sterilized aqueous preparation containing an active compound which is isotonic to the recipient's blood. In the case of an injection, for example, a solution for injection is prepared using a carrier comprising a saline solution, a glucose solution, or a mixture of a saline solution and a glucose solution.

The parenteral preparations may also comprise one or more auxiliary components selected from the excipients, disintegrators, lubricants, binders, surfactants and plasticizers described in the above description of oral preparations and diluents, antiseptics, flavors, etc.

The dose and the administration schedule of Compound (I) or (IA), prodrugs thereof, or pharmaceutically

acceptable salts thereof will vary depending upon the administration route, the age and body weight of a patient, and the nature and degree of severity of the symptom to be treated. In general, in the case of oral administration, the active ingredient is administered in a dose of 0.01 mg to 1 g, preferably 0.05 to 50 mg, per adult once to several times per day. In the case of parenteral administration such as intravenous administration, the active ingredient is administered in a dose of 0.001 to 500 mg, preferably 0.01 to 100 mg, per adult once to several times per day. However, the dose and the administration schedule may vary depending upon various conditions as given above.

Typical therapeutic effects for tumors by Compound (I) are illustrated below referring to Test Examples.

Test Example 1: Growth inhibition test on human chronic myelocytic leukemia K562 cells

One thousand cells of human chronic myelocytic leukemia K562 were inoculated into each well of a 96-well microplate (manufactured by Nunc Corp.), and using RPMI1640 medium (culture medium) containing 10% fetal calf serum (FCS), preculturing was performed in a 5% carbon dioxide incubator at 37°C for 24 hours. A dimethyl sulfoxide (DMSO) solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37°C for 72 hours. After completion of the culturing, 10 μL of WST-1 (4-[3-(4-Iodophenyl)-2-(4-nitrophenyl)-2H-5-tetrazolio]-1,3-benzene disulfonate) labeled mixture (manufactured by Roche Diagnostic Corp.) was added to each well, and culturing was performed in the 5% carbon dioxide incubator at 37°C for 2 hours. Using a microplate spectrophotometer (SpectraMax 340PC384; manufactured by Nihon Molecular Devices), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 3. As is evident from Table 3, the group of compounds tested as representative examples of Compound (I) exhibits cell growth inhibitory activity against human chronic myelocytic leukemia K562 cells at a concentration of 10 μmol/L. That is, it has been confirmed that Compound (I) is useful as a therapeutic agent for treating chronic myelocytic leukemia.

Test Example 2: Growth inhibition test on human acute myelocytic leukemia MV4;11 cells

Ten thousand cells of human acute myelocytic leukemia MV4;11 were inoculated into each well of a 96-well microplate (manufactured by Nunc Corp.), and using IMDM medium (culture medium) containing 10% FCS, preculturing was performed in a 5% carbon dioxide incubator at 37°C for 24 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37°C for 72 hours. After completion of the culturing, 10 μL of WST-1 labeled mixture (manufactured by Roche Diagnostic Corp.) was added to each well, and culturing was performed in the 5% carbon dioxide incubator at 37°C for 1 hour. Using a microplate spectrophotometer (SpectraMax 340PC384; manufactured by Nihon Molecular Devices), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 3. As is evident from Table 3, the group of compounds tested as representative examples of Compound (I) exhibits cell growth inhibitory activity against human acute myelocytic leukemia MV4;11 cells at a concentration of 10 μmol/L. That is, it has been confirmed that Compound (I) is useful as a therapeutic agent for treating acute myelocytic leukemia.

Test Example 3: Growth inhibition test on human multiple myeloma NCI-H929 cells

Ten thousand cells of human multiple myeloma NCI-H929 were inoculated into each well of a 96-well microplate (manufactured by Nunc Corp.), and using RPMI medium (culture medium) containing 10% FCS, preculturing was performed in a 5% carbon dioxide incubator at 37°C for 24 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37°C for 72 hours. After completion of the culturing, 10 μL of WST-1 labeled mixture (manufactured by Roche Diagnostic Corp.) was added to each well, and culturing was performed in the 5% carbon dioxide incubator at 37°C for 2 hours.

Using a microplate spectrophotometer (SpectraMax 340PC384; manufactured by Nihon Molecular Devices), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 3. As is evident from Table 3, the group of compounds tested as representative examples of Compound (I) exhibits cell growth inhibitory activity against human multiple myeloma NCI-H929 cells at a concentration of 10 μmol/L. That is, it has been confirmed that Compound (I) is useful as a therapeutic agent for treating multiple myeloma.

Test Example 4: Growth inhibition test on human T-cell lymphoma Karpas-299 cells

Five thousand cells of human T-cell lymphoma Karpas-299 were inoculated into each well of a 96-well microplate (manufactured by Nunc Corp.), and using RPMI medium (culture medium) containing 10% FCS, preculturing was performed in a 5% carbon dioxide incubator at 37˚C for 5 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37˚C for 72 hours. After completion of the culturing, 10 μL of WST-1 labeled mixture (manufactured by Roche Diagnostic Corp.) was added to each well, and culturing was performed in the 5% carbon dioxide incubator at 37˚C for 2 hours. Using a microplate spectrophotometer (SpectraMax 340PC384; manufactured by Nihon Molecular Devices), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 3. As is evident from Table 3, the group of compounds tested as representative examples of Compound (I) exhibits cell growth inhibitory activity against human T-cell lymphoma Karpas-299 cells at a concentration of 10 μmol/L. That is, it has been confirmed that Compound (I) is useful as a therapeutic agent for treating T-cell lymphoma.

Test Example 5: Growth inhibition test on human chronic lymphocytic leukemia-derived MEC-1 cells

Ten thousand cells of human chronic lymphocytic leukemia-derived MEC-1 were inoculated into each well of a 96-well microplate (manufactured by Nunc Corp.), and using RPMI1640 medium (culture medium) containing 10% FCS, preculturing was performed in a 5% carbon dioxide incubator at 37˚C for 1 hour. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37˚C for 72 hours. After completion of the culturing, 10 μL of WST-1 labeled mixture (manufactured by Roche Diagnostic Corp.) was added to each well, and culturing was performed in the 5% carbon dioxide incubator at 37˚C for 3 hours. Using a microplate spectrophotometer (M-SPmax 250; manufactured by Molecular Devices Corp.), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 3. As is evident from Table 3, the group of compounds tested as representative examples of Compound (I) exhibits cell growth inhibitory activity against human chronic lymphocytic leukemia-derived MEC-1 cells at a concentration of 10 μmol/L. That is, it has been confirmed that Compound (I) is useful as a therapeutic agent for treating chronic lymphocytic leukemia.

[Table 3]

| Comp ound | Viability of tumor cells(% viability) | | | | |
|---|---|---|---|---|---|
| | Human chronic myelocytic leukemia K562 cells | Human acute myelocytic leukemia MV4;11 cells | Human chronic lymphocytic leukemia MEC-1 cells | Human multiple myeloma NCI-H929 cells | Human T-cell lymphoma Karpas-299 cells |
| 13 | 26 | 0 | 7 | 0 | 27 |
| 16 | 4 | 0 | 3 | 0 | 13 |
| 17 | 4 | 0 | 4 | 0 | 16 |
| 32 | 4 | 0 | 4 | 0 | 15 |
| 33 | 4 | 0 | 4 | 0 | 14 |
| 38 | 4 | 0 | 4 | 0 | 14 |
| 41 | 4 | 0 | 4 | 0 | 19 |
| 56 | 5 | 0 | 4 | 0 | 14 |
| 61 | 5 | 0 | 5 | 0 | 13 |
| 69 | 4 | 0 | 4 | 0 | 14 |
| 88 | 4 | 0 | 5 | 0 | 16 |
| 91 | 4 | 0 | 4 | 0 | 16 |
| 95 | 4 | 0 | 4 | 0 | 15 |
| 106 | 4 | 0 | 4 | 0 | 13 |
| 107 | 5 | 0 | 5 | 0 | 14 |
| 122 | 4 | 0 | 4 | 0 | 12 |
| 124 | 4 | 0 | 4 | 0 | 14 |
| 125 | 4 | 0 | 5 | 0 | 15 |
| 134 | 4 | 0 | 6 | 0 | 16 |
| 139 | 4 | 1 | 5 | 0 | 14 |
| 144 | 4 | 0 | 4 | 0 | 15 |
| 149 | 4 | 0 | 5 | 0 | 14 |
| 151 | 4 | 0 | 4 | 0 | 13 |

Also, from the results of Test Examples 1 to 5, it has been confirmed that Compound (I) is useful as a therapeutic agent for hematopoietic tumors such as leukemia, lymphoma, and multiple myeloma.

Test Example 6: Growth inhibition test on human mammary cancer BT-474 cells

Four thousand cells of human mammary cancer BT-474 were inoculated into each well of a 96-well microplate (manufactured by Nunc Corp.), and using Dulbecco's Modified Eagle's Medium (DMEM) (culture medium) containing 10% FCS, preculturing was performed in a 5% carbon dioxide incubator at 37˚C for 24 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37˚C for 72 hours. After completion of the culturing, 10 μL of WST-1 labeled mixture (manufactured by Roche Diagnostic Corp.) was added to each well, and culturing was performed in the 5% carbon dioxide incubator at 37˚C for 2 hours. Using a microplate spectrophotometer (SpectraMax 340PC384; manufactured by Nihon Molecular Devices), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells

was designated as 0%. By comparing these values with the absorbance difference obtained at the well in which each test compound was added, the cell viability (% viability) after treatment with the test compound was calculated.

The results thereof are shown in Table 4. As is evident from Table 4, the group of compounds tested as representative examples of Compound (I) exhibits cell growth inhibitory activity against human mammary cancer BT-474 cells at a concentration of 10 μmol/L. That is, it has been confirmed that Compound (I) is useful as a therapeutic agent for treating mammary cancer.

Test Example 7: Growth inhibition test on human lung cancer NCI-H596 cells

Four thousand cells of human lung cancer NCI-H596 were inoculated into each well of a 96-well microplate (manufactured by Nunc Corp.), and using RPMI1640 medium (culture medium) containing 10% FCS, preculturing was performed in a 5% carbon dioxide incubator at 37˚C for 24 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37˚C for 72 hours. After completion of the culturing, 10 μL of WST-1 labeled mixture (manufactured by Roche Diagnostic Corp.) was added to each well, and culturing was performed in the 5% carbon dioxide incubator at 37˚C for 2 hours. Using a microplate spectrophotometer (Model 550; manufactured by Bio-Rad), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 4. As is evident from Table 4, the group of compounds tested as representative examples of Compound (I) exhibits cell growth inhibitory activity against human lung cancer NCI-H596 cells at a concentration of 10 μmol/L. That is, it has been confirmed that Compound (I) is useful as a therapeutic agent for treating lung cancer.

Test Example 8: Growth inhibition test on human renal cancer OS-RC-2 cells

One thousand cells of human renal cancer OS-RC-2 were inoculated into each well of a 96-well microplate (manufactured by Nunc Corp.), and using RPMI1640 medium (culture medium) containing 10% FCS, preculturing was performed in a 5% carbon dioxide incubator at 37˚C for 24 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37˚ C for 72 hours. After completion of the culturing, 10 μL of WST-1 labeled mixture (manufactured by Roche Diagnostic Corp.) was added to each well, and culturing was performed in the 5% carbon dioxide incubator at 37˚C for 2 hours. Using a microplate spectrophotometer (Model 550; manufactured by Bio-Rad), the absorbance of each well was measured at 450 nm and 655 nm. The value obtained by subtracting the absorbance at 450 nm from the absorbance at 655 nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 4. As is evident from Table 4, the group of compounds tested as representative examples of Compound (I) exhibit cell growth inhibitory activity against human renal cancer OS-RC-2 cells at a concentration of 10 μmol/L. That is, it has been confirmed that Compound (I) is useful as a therapeutic agent for treating renal cancer.

Test Example 9: Growth inhibition test on human prostate cancer 22Rv1 cells

Five thousand cells of human prostate cancer 22Rv1 were inoculated into each well of a 96-well microplate (manufactured by Nunc Corp.), and using RPMI1640 medium (culture medium) containing 10% FCS, preculturing was performed in a 5% carbon dioxide incubator at 37˚C for 24 hours. A DMSO solution of each test compound prepared in a concentration of 10 mmol/L was diluted with the culture medium to a final concentration of 10 μmol/L, and the diluted solution was added to each well. The individual wells were further cultured in the 5% carbon dioxide incubator at 37˚ C for 72 hours. After completion of the culturing, 10 μL of WST-1 labeled mixture (manufactured by Roche Diagnostic Corp.) was added to each well, and culturing was performed in the 5% carbon dioxide incubator at 37˚C for 2 hours. Using a microplate spectrophotometer (Model 550; manufactured by Bio-Rad), the absorbance of each well was measured at 450 nm and 655 nm. The value obtained by subtracting the absorbance at 450 nm from the absorbance at 655

nm (absorbance difference) was calculated for each well. The value for cells not incorporated with a test compound was designated as 100%, and the value at a well not containing cells was designated as 0%. By comparing these values with the absorbance difference obtained at the well in which each test compound was added, the cell viability (% viability) after treatment with the test compound was calculated.

The results thereof are shown in Table 4. As is evident from Table 4, the group of compounds tested as representative examples of Compound (I) exhibits cell growth inhibitory activity against human prostate cancer 22Rv1 cells at a concentration of 10 μmol/L. That is, it has been confirmed that Compound (I) is useful as a therapeutic agent for treating prostate cancer.

[Table 4]

| Compound | Viability of tumor cells(% viability) | | | |
|---|---|---|---|---|
| | Human mammary cancer BT-474 cells | Human lung cancer NCI-H596 cells | Human renal cancer OS-RC-2 cells | Human prostate cancer 22Rv1 cells |
| 13 | 12 | 35 | 66 | 6 |
| 16 | 12 | 19 | 17 | 5 |
| 17 | 10 | 16 | 20 | 5 |
| 32 | 13 | 15 | 17 | 5 |
| 33 | 13 | 20 | 18 | 5 |
| 38 | 13 | 15 | 18 | 4 |
| 41 | 14 | 12 | 18 | 5 |
| 56 | 13 | 20 | 18 | 5 |
| 61 | 12 | 25 | 25 | 5 |
| 69 | 13 | 15 | 16 | 5 |
| 88 | 13 | 14 | 17 | 6 |
| 91 | 13 | 14 | 17 | 5 |
| 95 | 13 | 14 | 18 | 5 |
| 106 | 12 | 12 | 16 | 5 |
| 107 | 13 | 16 | 18 | 5 |
| 122 | 12 | 15 | 15 | 5 |
| 124 | 13 | 15 | 15 | 5 |
| 125 | 14 | 17 | 20 | 5 |
| 134 | 13 | 17 | 21 | 6 |
| 139 | 14 | 16 | 21 | 5 |
| 144 | 13 | 14 | 16 | 5 |
| 149 | 14 | 14 | 20 | 4 |
| 151 | 13 | 28 | 30 | 5 |

Also, from the results of Test Examples 6 to 9, it has been confirmed that Compound (I) is useful as a therapeutic agent for solid tumors such as mammary cancer, lung cancer, renal cancer and prostate cancer.

<u>Test Example 10: Antitumor effect in vivo using mouse model transplanted with human chronic myelocytic leukemia K562 cells</u>

Using an experimental system in which human chronic myelocytic leukemia K562 cells were transplanted into immunodeficient mice as disease models of hematopoietic tumor, the antitumor effect in vivo of Compound 33 was examined.

One day before the transplantation of cancer cells, an anti-asialo GM1 antibody was intraabdominally administered to Fox C.B-17/Icr-scidJc1 mice (CLEA Japan) in an amount of 0.3 mg per mouse. K562 cells were cultured and grown in RPMI1640 medium containing 10% fetal calf serum (FCS) in a 5% carbon dioxide incubator at 37˚C, and the cultured cells ($1 \times 10^7$ cells/mouse) were subcutaneously, ventrally transplanted into the mice. Ten days after the transplantation, the major axis and minor axis of the tumors subcutaneously grown were measured with slide calipers, and the tumor volume was determined according to the following formula:

[Formula 1]

$$\text{Tumor Volume} \quad V\,(\text{mm}^3) = \frac{\text{major axis (mm)} \times [\text{ minor axis (mm) }]^2}{2}$$

At the same time, the body weight of each mouse was measured, and the mice were divided into two groups, i.e., a group to be administered with drugs and a group not to be administered with drugs, such that each group consists of five mice with various weights and tumor volumes. This day was defined as day 0 of the administration test. Drug administration was started in the following manner.

Compound 33 was dissolved in a solvent for administration [a solution in which N,N-dimethylacetamide (manufactured by Wako Pure Chemical Industries, Ltd.), CREMOPHOR EL (manufactured by Sigma-Aldrich Co.), and physiological saline (manufactured by Otsuka Pharmaceutical Co., Ltd.) were mixed at a volume ratio of 1:1:8] at a concentration of 10 mg/mL. The resulting solution was intravenously administered from the caudal vein to each mouse in a dose of 0.01 mL per gram of the body weight of the mouse (100 mg/kg) twice a day on days 0, 1, 2, 7, 8, and 9 after the start of administration. The tumor volume of each of the group not administered with a drug and the group administered with Compound 33 was measured on 4, 7, 10, 14, 17, and 22 days after the start of the administration test.

The results thereof are shown in Fig. 1. In the group administered with Compound 33, apparent suppression of tumor growth is observed, and it is found that Compound 33 has the antitumor effect, also in vivo, on the mice transplanted with human chronic myelocytic leukemia K562 cells. As a result, it has been confirmed that, by the administration of Compound (I), the therapeutic effect on hematopoietic tumor is obtained also in vivo.

<u>Test Example 11: Antitumor effect in vivo using mouse model transplanted with human lung cancer NCI-H596 cells</u>

Using an experimental system in which human lung cancer NCI-H596 cells were transplanted into immunodeficient mice as disease models of solid tumor, the antitumor effect in vivo of Compound 33 was examined.

NCI-H596 cells were cultured and grown in RPMI1640 medium containing 10% fetal calf serum (FCS) in a 5% carbon dioxide incubator at 37˚C, and the cultured cells ($1 \times 10^7$ cells/mouse) were subcutaneously, ventrally transplanted into BALB/cAJc1-nu mice (CLEA Japan). From the mice in which tumors were formed, the tumors were removed. The tumor tissues were cut into small pieces of about 8 $mm^3$, which were subcutaneously, ventrally transplanted, using a trocar needle, into BALB/cAJc1-nu mice (CLEA Japan) to be used for experiment. Seventeen days after the transplantation, the major axis and minor axis of the tumors subcutaneously grown were measured with slide calipers, and the tumor volume was determined according to the following formula:

[Formula 2]

$$\text{Tumor Volume} \quad V\,(\text{mm}^3) = \frac{\text{major axis (mm)} \times [\text{ minor axis (mm) }]^2}{2}$$

At the same time, the body weight of each mouse was measured, and the mice were divided into two groups, i.e., a group to be administered with drugs and a group not to be administered with drugs, such that each group consists of five mice with various weights and tumor volumes. This day was defined as day 0 of the administration test. Drug administration was started in the following manner.

Compound 33 was dissolved in a solvent for administration [a solution in which N,N-dimethylacetamide (manufactured by Wako Pure Chemical Industries, Ltd.), CREMOPHOR EL (manufactured by Sigma-Aldrich Co.), and physiological saline (manufactured by Otsuka Pharmaceutical Co., Ltd.) were mixed at a volume ratio of 1:1:8] at a concentration of 5 mg/mL. The resulting solution was intravenously administered from the caudal vein to each mouse in a dose of 0.01 mL per gram of the body weight of the mouse (50 mg/kg) twice a day on days 0 to 4 consecutively after the start

of administration. The tumor volume of each of the group not administered with a drug and the group administered with Compound 33 was measured on 4, 7, 10, 14, and 17 days after the start of the administration test.

The results thereof are shown in Fig. 2. In the group administered with Compound 33, apparent suppression of tumor growth is observed, and it is found that Compound 33 has the antitumor effect, also in vivo, on the mice transplanted with human lung cancer NCI-H596 cells. As a result, it has been confirmed that, by the administration of Compound (I), the therapeutic effect on solid tumor is obtained also in vivo.

Test Example 12: Antitumor effect in vivo using mouse model transplanted with human prostate cancer 22Rv1 cells

Using an experimental system in which human prostate cancer 22Rv1 cells were transplanted into immunodeficient mice as disease models of solid tumor, the antitumor effect in vivo of Compound 33 was examined.

22Rv1 cells were cultured and grown in RPMI1640 medium containing 10% fetal calf serum (FCS) in a 5% carbon dioxide incubator at 37°C, and the cultured cells ($1\times10^7$ cells/mouse) were subcutaneously, ventrally transplanted into BALB/cAJc1-nu mice (CLEA Japan). Seventeen days after the transplantation, the major axis and minor axis of the tumors subcutaneously grown were measured with slide calipers, and the tumor volume was determined according to the following formula:

[Formula 3]

$$\text{Tumor Volume} \quad V\,(mm^3) = \frac{\text{major axis (mm)} \times [\text{ minor axis (mm) }]^2}{2}$$

At the same time, the body weight of each mouse was measured, and the mice were divided into two groups, i.e., a group to be administered with drugs and a group not to be administered with drugs, such that each group consists of five mice with various weights and tumor volumes. This day was defined as day 0 of the administration test. Drug administration was started in the following manner.

Compound 33 was dissolved in a solvent for administration [a solution in which N,N-dimethylacetamide (manufactured by Wako Pure Chemical Industries, Ltd.), CREMOPHOR EL (manufactured by Sigma-Aldrich Co.), and physiological saline (manufactured by Otsuka Pharmaceutical Co., Ltd.) were mixed at a volume ratio of 1:1:8] at a concentration of 10 mg/mL. The resulting solution was intravenously administered from the caudal vein to each mouse in a dose of 0.01 mL per gram of the body weight of the mouse (100 mg/kg) twice a day on days 0 to 4 consecutively after the start of administration. The tumor volume of each of the group not administered with a drug and the group administered with Compound 33 was measured on 4, 7, 10, 14, and 17 days after the start of the administration test.

The results thereof are shown in Fig. 3. In the group administered with Compound 33, apparent suppression of tumor growth is observed, and it is found that Compound 33 has the antitumor effect, also in vivo, on the mice transplanted with human prostate cancer 22Rv1 cells. As a result, it has been confirmed that, by the administration of Compound (I), the therapeutic effect on solid tumor is obtained also in vivo.

Example 1

A tablet including the following composition is prepared by a conventional process. Compound 4 (40 g), lactose (286.8 g) and corn starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Clean Press Correct 12, Kikusui Co.).

Prescription

| | |
|---|---|
| Compound 4 | 20 mg |
| Lactose | 143.4 mg |
| Corn starch | 30 mg |
| Hydroxypropylcellulose | 6 mg |

(continued)

| | |
|---|---|
| Magnesium stearate | 0.6 mg |
| | 200 mg |

Example 2

A tablet including the following composition is prepared by a conventional process. Compound 6 (40 g), lactose (286.8 g) and corn starch (60 g) are mixed, followed by adding 10% hydroxypropylcellulose aqueous solution (120 g) thereto. After the resulting mixture is kneaded, granulated, and dried according to a conventional process, the size of the granules is prepared for tablet pressing. The granules are mixed with magnesium stearate (1.2 g) and then pressed to make tablets (each tablet containing 20 mg of the active ingredient) by a tablet making machine having a striker of 8 mm diameter (Clean Press Correct 12, Kikusui Co.).

Prescription

| | |
|---|---|
| Compound 6 | 20 mg |
| Lactose | 143.4 mg |
| Corn starch | 30 mg |
| Hydroxypropylcellulose | 6 mg |
| Magnesium stearate | 0.6 mg |
| | 200 mg |

Example 3

An injection including the following composition is prepared by a conventional process. Compound 7 (1 g) and sodium chloride (9 g) are dissolved in injectable distilled water to make the total volume to 1000 mL. The resulting solution is filtered with a 0.2 μm disposable membrane filter under sterile condition and is dispensed into glass vials at a volume of 2 mL per vial (each vial contains 2 mg of the active ingredient) under the sterile condition to obtain the injections.

Prescription

| | |
|---|---|
| Compound 7 | 2 mg |
| Sodium Chloride | 18 mg |
| Injectable distilled water | proper amount |
| | 2.00 mL |

Industrial Applicability

The present invention provides a therapeutic agent for a tumor selected from a hematopoietic tumor and a solid tumor comprising, as an active ingredient, a benzoyl compound, a prodrug thereof or a pharmaceutically acceptable salt thereof.

**Claims**

1. A therapeutic agent for a tumor selected from a hematopoietic tumor and a solid tumor which comprises, as an active ingredient, a benzoyl compound represented by General Formula (I):

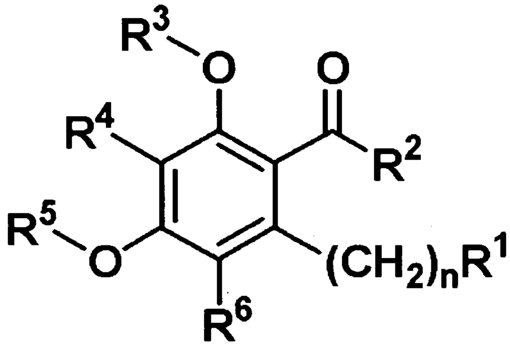

( I )

[wherein
n represents an integer of 1 to 5;
$R^1$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocyclic alkyl, substituted or unsubstituted aryl,
$CONR^7R^8$ (wherein $R^7$ and $R^8$ may be the same or different, and each represents a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocyclic alkyl, or substituted or unsubstituted aroyl, or $R^7$ and $R^8$ are combined together with the adjacent nitrogen atom thereto to form a substituted or unsubstituted heterocyclic group), or
$NR^9R^{10}$ (wherein $R^9$ and $R^{10}$ have the same meanings as the above $R^7$ and $R^8$, respectively);
$R^2$
represents substituted or unsubstituted aryl,
or
a substituted or unsubstituted aromatic heterocyclic group;
$R^3$ and $R^5$
may be the same or different, and each represents a hydrogen atom,
substituted or unsubstituted lower alkyl,
substituted or unsubstituted lower alkenyl,
substituted or unsubstituted lower alkanoyl,
substituted or unsubstituted cycloalkyl,
substituted or unsubstituted aralkyl,
or
substituted or unsubstituted aroyl;
$R^4$ represents a hydrogen atom, hydroxy,
or
halogen; and
$R^6$ represents a hydrogen atom,
halogen,
cyano,
nitro,
substituted or unsubstituted lower alkyl,
substituted or unsubstituted lower alkenyl,
substituted or unsubstituted lower alkynyl,
substituted or unsubstituted lower alkoxy,
substituted or unsubstituted cycloalkyl,
amino,
lower alkylamino,
di-lower alkylamino,
carboxy,
substituted or unsubstituted lower alkoxycarbonyl,

substituted or unsubstituted lower alkanoyl,
substituted or unsubstituted aryloxy,
substituted or unsubstituted aryl,
a substituted or unsubstituted heterocyclic group,
substituted or unsubstituted aralkyl,
or
substituted or unsubstituted heterocyclic alkyl; with the proviso that

(i) when $R^3$ and $R^5$ are methyl and $R^4$ and $R^6$ are hydrogen atoms, and

(a) when $-(CH_2)_nR^1$ is methoxycarbonylmethyl,
$R^2$ is not a group selected from 2,4,6-trimethoxy-5-methoxycarbonyl-3-nitrophenyl, 3-cyano-2,4,6-trimethoxyphenyl, 5-cyano-2-ethoxy-4,6-dimethoxy-3-nitrophenyl, 2,6-dimethoxyphenyl, 2-chloro-6-methoxyphenyl and 2-chloro-4,6-dimethoxy-5-methoxycarbonyl-3-nitrophenyl,
(b) when $-(CH_2)_nR^1$ is ethoxycarbonylmethyl,
$R^2$ is not 2,4,6-trimethoxy-3-methoxycarbonylphenyl, and
(c) when $-(CH_2)_nR^1$ is N,N-dimethylaminomethyl,
$R^2$ is not phenyl,

(ii) when $R^3$, $R^4$, $R^5$ and $R^6$ are hydrogen atoms, and

(a) when $-(CH_2)_nR^1$ is 2-(acetoxymethyl)heptyl, 3-oxopentyl or pentyl,
$R^2$ is not 6-hydroxy-4-methoxy-3-methoxycarbonyl-2-pentylphenyl,
(b) when $-(CH_2)_nR^1$ is 3-oxopentyl,
$R^2$ is not a group selected from 3-benzyloxycarbonyl-6-hydroxy-4-methoxy-2-pentylphenyl and 3-carboxy-6-hydroxy-4-methoxy-2-pentylphenyl, and
(c) when $-(CH_2)_nR^1$ is n-propyl,
$R^2$ is not 2,4-dihydroxy-6-[(4-hydroxy-2-oxopyran-6-yl)methyl]phenyl,

(iii) when $R^3$ and $R^4$ are hydrogen atoms, $R^5$ is methyl, $R^6$ is methoxycarbonyl and $-(CH_2)_nR^1$ is pentyl,
$R^2$ is not a group selected from 6-[2-(acetoxymethyl)heptyl]-2,4-dihydroxyphenyl, 2,4-dihyroxy-6-pentylphenyl and 2,4-dihydroxy-6-(3-oxopentyl)phenyl,
(iv) when $R^3$ and $R^5$ are benzyl, $R^4$ and $R^6$ are hydrogen atoms, and $-(CH_2)_nR^1$ is 3-oxopentyl,
$R^2$ is not a group selected from 6-benzyloxy-4-methoxy-3-methoxycarbonyl-2-pentylphenyl and 6-benzyloxy-3-benzyloxycarbonyl-4-methoxy-2-pentylphenyl,
(v) when $R^3$ is benzyl, $R^4$ is a hydrogen atom, $R^5$ is methyl, $-(CH_2)_nR^1$ is pentyl and $R^6$ is methoxycarbonyl or benzyloxycarbonyl,
$R^2$ is not 2,4-bis(benzyloxy)-6-(3-oxopentyl)phenyl,
(vi) when $R^3$ and $R^4$ are hydrogen atoms, $R^5$ is methyl, $-(CH_2)_nR^1$ is pentyl, and $R^6$ is carboxy or benzyloxycarbonyl,
$R^2$ is not 2,4-dihydroxy-6-(3-oxopentyl)phenyl, and
(vii) when $R^3$, $R^4$, and $R^6$ are hydrogen atoms, $R^5$ is n-propyl, and $-(CH_2)_nR^1$ is 5-(1,1-dimetylpropyl)-4-(2-hydrobenzotriazol-2-yl)-2-hydroxyphenylmethyl,
$R^2$ is not phenyl],

a prodrug thereof or a pharmaceutically acceptable salt thereof.

2. The therapeutic agent for a tumor according to Claim 1, wherein $R^2$ is a substituted or unsubstituted aromatic heterocyclic group, aryl substituted with 1-3 substituents or aryl.

3. The therapeutic agent for a tumor according to Claim 1, wherein $R^2$ is aryl substituted with 1-3 substituents or aryl.

4. The therapeutic agent for a tumor according to Claim 1, wherein $R^2$ is phenyl substituted with 1-3 substituents or phenyl.

5. The therapeutic agent for a tumor according to Claim 1, wherein $R^2$ is a substituted or unsubstituted aromatic heterocyclic group.

**6.** The therapeutic agent for a tumor according to any of Claims 1 to 5, wherein $R^3$ and $R^5$ may be the same or different, and each is a hydrogen atom, substituted or unsubstituted lower alkyl, substituted or unsubstituted lower alkanoyl, substituted or unsubstituted aroyl or substituted or unsubstituted lower alkenyl.

**7.** The therapeutic agent for a tumor according to any of Claims 1 to 5, wherein $R^3$ , $R^4$, and $R^5$ each are hydrogen atoms.

**8.** The therapeutic agent for a tumor according to any of Claims 1 to 7, wherein $R^1$ is $CONR^7R^8$ (wherein $R^7$ and $R^8$ have the same meanings as defined above, respectively).

**9.** The therapeutic agent for a tumor according to any of Claims 1 to 7, wherein $R^1$ is $CONR^{7A}R^{8B}$ (wherein $R^{7A}$ and $R^{8A}$ may be the same or different, and each represents a hydrogen atom, substituted or unsubstituted lower alkyl, or substituted or unsubstituted heterocyclic alkyl).

**10.** The therapeutic agent for a tumor according to any of Claims 1 to 7, wherein $R^1$ is $CONR^{7B}R^{8B}$ (wherein $R^{7B}$ and $R^{8B}$ are combined together with the adjacent nitrogen atom thereto to form a substituted or unsubstituted heterocyclic group).

**11.** The therapeutic agent for a tumor according to any of Claims 1 to 7, wherein $R^1$ is substituted or unsubstituted lower alkoxy.

**12.** The therapeutic agent for a tumor according to any of Claims 1 to 11, wherein $R^6$ is a hydrogen atom, lower alkyl, halogen or aryl.

**13.** The therapeutic agent for a tumor according to any of Claims 1 to 11, wherein $R^6$ is lower alkyl.

**14.** The therapeutic agent for a tumor according to any of Claims 1 to 11, wherein $R^6$ is ethyl.

**15.** The therapeutic agent for a tumor according to any of Claims 1 to 14, wherein the tumor is a hematopoietic tumor.

**16.** The therapeutic agent for a tumor according to Claim 15, wherein the hematopoietic tumor is a tumor selected from leukemia, multiple myeloma and lymphoma.

**17.** The therapeutic agent for a tumor according to any of Claims 1 to 14, wherein the tumor is a solid tumor.

**18.** The therapeutic agent for a tumor according to Claim 17, wherein the solid tumor is a tumor selected from colon cancer, esophageal cancer, gastric cancer, hepatic cancer, pancreatic cancer, biliary tract cancer, bladder cancer, renal cancer, prostatic cancer, mammary cancer, uterine cervix cancer, uterine body cancer, ovarian cancer, head and neck cancer, lung cancer, osteosarcoma, melanoma, and brain tumor.

**19.** A method for treating a tumor selected from a hematopoietic tumor and a solid tumor, comprising administering an effective amount of a benzoyl compound represented by General Formula (I) described in Claim 1, a prodrug thereof or a pharmaceutically acceptable salt thereof.

**20.** Use of a benzoyl compound represented by General Formula (I) described in Claim 1, a prodrug thereof or a pharmaceutically acceptable salt thereof for the manufacture of a therapeutic agent for a tumor selected from a hematopoietic tumor and a solid tumor.

[Figure 1]

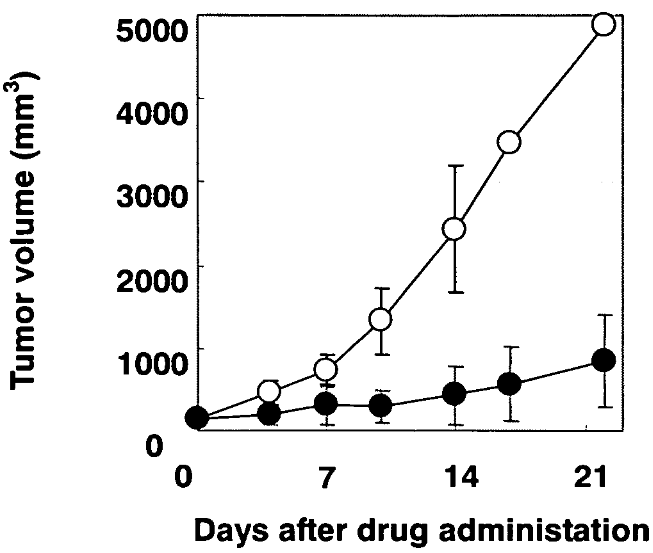


[Figure 2]

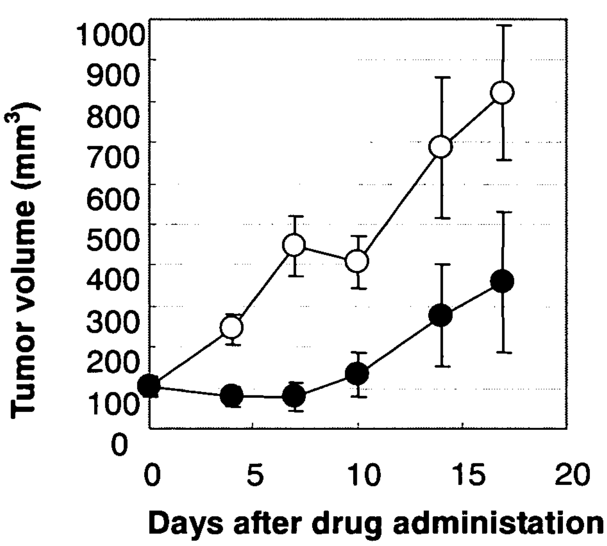

[Figure 3]

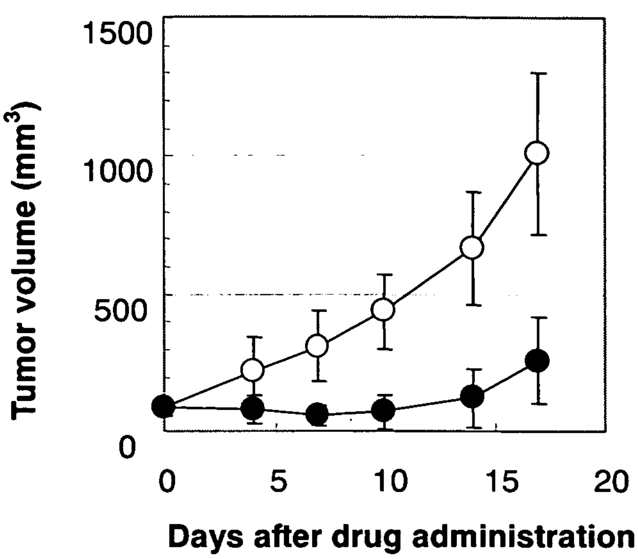
1500
1000
500
0
Tumor volume (mm3)
0
5
10
15
20
Days after drug administration

**INTERNATIONAL SEARCH REPORT**

International application No.
PCT/JP2006/302996

A. CLASSIFICATION OF SUBJECT MATTER
***A61K31/12***(2006.01), ***A61K31/165***(2006.01), ***A61K31/216***(2006.01), ***A61K31/343***(2006.01), ***A61K31/36***(2006.01), ***A61K31/381***(2006.01), ***A61K31/40***(2006.01), ***A61K31/4015***(2006.01), ***A61K31/4406***(2006.01), ***A61K31/445***(2006.01),
According to International Patent Classification (IPC) or to both national classification and IPC

B. FIELDS SEARCHED
Minimum documentation searched (classification system followed by classification symbols)
A61K31/12, A61K31/165, A61K31/216, A61K31/343, A61K31/36, A61K31/381, A61K31/40, A61K31/4015, A61K31/4406, A61K31/445, A61K31/4535, A61K31/472, A61K31/495, A61K31/496, A61K31/506, A61K31/5375, A61K31/5377, A61P35/00,

Documentation searched other than minimum documentation to the extent that such documents are included in the fields searched

Electronic data base consulted during the international search (name of data base and, where practicable, search terms used)
CAplus(STN), REGISTRY(STN), EMBASE(STN), MEDLINE(STN), BIOSIS(STN)

C. DOCUMENTS CONSIDERED TO BE RELEVANT

| Category* | Citation of document, with indication, where appropriate, of the relevant passages | Relevant to claim No. |
|---|---|---|
| Y | WO 2005/000778 A1 (KYOWA HAKKO KOGYO KABUSHIKI KAISHA JP), 06 January, 2005 (06.01.05), Full text; particularly, Claims 11 to 24; pages 2, 39, lines 18 to 37; test example 1 (Family: none) | 1-18,20 |
| Y | Len N., Hsp90 inhibitors as novel cancer chemotherapeutic agents, Trends in molecular medicine, 2002, Vol.8, No. 4 (Suppl.), page s55 to s61., full text; particularly, page 56; Fig. 1; page 56, left column, line 22 to right column, line 5; page 57; Box 1 | 1-18,20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 May, 2006 (16.05.06) | 23 May, 2006 (23.05.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No. PCT/JP2006/302996

C (Continuation). DOCUMENTS CONSIDERED TO BE RELEVANT

| Category* | Citation of document, with indication, where appropriate, of the relevant passages | Relevant to claim No. |
|---|---|---|
| Y | Shiro S., Leonard M.N., Theodor W.S., et.al., KF25706, a novel oxime derivative of radicinol, exhibits in vivo antitumor activity via selective depletion of Hsp90 binding signaling molecules, Cancer research, 1999, Vol.59, No. 12, page 2931 to 2938, full text, page 2933, table 1; page 2936; Fig. 5, table 2 | 1-18,20 |
| Y | Georgios V.G., Yang L., Anas Y., The geldanamycin analogue heat shock protein 90 (HSP90) inhibitor 17-AAG in lymphomas: Molecular mechanisms of 17-AAG-induced cell death in Hodgkin disease and non-Hodgkin lymphoma cell lines, Blood, 2004, Vol.104, No.11, Part 1, page 899A., fullt text | 1-18,20 |
| Y | Lydia C., Pascale F., Daniela O., Denis G., Hsp90 Inhibitors induce apoptosis in human leukemia cells, Blood, 2004, Vol.104, No.11, Part 1, page 30a., full text | 1-18,20 |
| Y | WO 2003/055860 A1 (VERNALIS CAMBRIDGE LTD., GB), 10 July, 2003 (10.07.03), Full text; particularly, page 253, talbe 4; page 256, table 6 & EP 1456180 A1 & JP 2005-517675 A & US 2005-222230 A1 | 1-18,20 |

Form PCT/ISA/210 (continuation of second sheet) (April 2005)

**INTERNATIONAL SEARCH REPORT**

International application No.
PCT/JP2006/302996

Continuation of A. CLASSIFICATION OF SUBJECT MATTER
(International Patent Classification (IPC))

***A61K31/4535***(2006.01), ***A61K31/472***(2006.01), ***A61K31/495***(2006.01), ***A61K31/496***(2006.01), ***A61K31/506***(2006.01), ***A61K31/5375***(2006.01), ***A61K31/5377***(2006.01), ***A61P35/00***(2006.01), ***A61P35/02***(2006.01), ***C07D207/09***(2006.01), ***C07D207/12***(2006.01), ***C07D207/27***(2006.01), ***C07D211/22***(2006.01), ***C07D211/42***(2006.01), ***C07D211/52***(2006.01), ***C07D211/54***(2006.01), ***C07D211/60***(2006.01), ***C07D211/62***(2006.01), ***C07D213/40***(2006.01), ***C07D217/06***(2006.01), ***C07D239/42***(2006.01), ***C07D241/08***(2006.01), ***C07D295/08***(2006.01), ***C07D295/10***(2006.01), ***C07D295/12***(2006.01), ***C07D307/14***(2006.01), ***C07D317/54***(2006.01), ***C07D333/22***(2006.01), ***C07D409/10***(2006.01)

(According to International Patent Classification (IPC) or to both national classification and IPC)

Continuation of B. FIELDS SEARCHED
Minimum documentation searched (International Patent Classification (IPC))

A61P35/02, C07D207/09, C07D207/12, C07D207/27, C07D211/22, C07D211/42, C07D211/52, C07D211/54, C07D211/60, C07D211/62, C07D213/40, C07D217/06, C07D239/42, C07D241/08, C07D295/08, C07D295/10, C07D295/12, C07D307/14, C07D317/54, C07D333/22, C07D409/10

Minimum documentation searched (classification system followed by classification symbols)

Form PCT/ISA/210 (extra sheet) (April 2005)

**INTERNATIONAL SEARCH REPORT**

International application No.
PCT/JP2006/302996

**Box No. II Observations where certain claims were found unsearchable (Continuation of item 2 of first sheet)**

This international search report has not been established in respect of certain claims under Article 17(2)(a) for the following reasons:

1. [x] Claims Nos.: 19
because they relate to subject matter not required to be searched by this Authority, namely:
Claim 19 is relevant to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200181288 A
- WO 2005000778 A


### Non-patent literature cited in the description


- *J. Med. Chem.,* 1997, vol. 40, 2011-2016
- *Drug Dev. Res.,* 1995, vol. 34, 220-230
- *Advances in Drug Res.,* 1984, vol. 13, 224-331
- **BUNDGAARD.** Design of Prodrugs. Elsevier Press, 1985
- **T.W. GREENE.** Protective Groups in Organic Synthesis. John Wiley & Sons Inc, 1999